# EUROPEAN PATENT SPECIFICATION

(11) **EP 4 547 187 B1**
(45) Date of publication and mention of the grant of the patent: **02.12.2026**
(21) Application number: 23744995.4
(22) Date of filing: 27.06.2023
(51) Int. Cl.: A61F 13/496, A61F 13/49, A61F 13/551

(54) **ABSORBENT ARTICLES WITH FRANGIBLE PATHWAYS HAVING TEAR ZONES**
ABSORBIERENDE ARTIKEL MIT ZERBRECHLICHEN PFADEN MIT REISSBEREICHEN
ARTICLES ABSORBANTS À VOIES FRANGIBLES AYANT DES ZONES DE DÉCHIRURE

(30) Priority: 30.06.2022 US 202263357043 P; 14.12.2022 US 202263432400 P; 14.12.2022 US 202263432401 P; 14.12.2022 US 202263432402 P; 14.12.2022 US 202263432403 P; 14.12.2022 US 202263432404 P; 14.12.2022 US 202263432406 P; 14.12.2022 US 202263432410 P; 14.12.2022 US 202263432413 P
(43) Date of publication of application: 07.05.2025
(60) Divisional application: 26213737.5
(73) Proprietor: The Procter & Gamble Company, Cincinnati, OH 45202 (US)
(72) Inventor: XU, Han, Cincinnati, Ohio 45202 (US); TAYLOR, Nicholas Alexander, Cincinnati, Ohio 45202 (US); WILLHAUS, Keith Richard, Cincinnati, Ohio 45202 (US); RAYCHECK, Jeromy Thomas, Cincinnati, Ohio 45202 (US)
(74) Representative: P&G Patent Germany
(86) International application number: PCT/US2023/069126
(87) International publication number: WO 2024/006728

(56) References cited:
- US-B2- 8 043 274
- US-B2- 8 192 417

## Description

### FIELD

The present disclosure relates to absorbent articles, and more particularly, to absorbent articles having front and/or back waist regions including one or more frangible pathways.

### BACKGROUND

Some absorbent articles have components that include elastomeric laminates. Such elastomeric laminates may include an elastic material bonded to one or more nonwovens. The elastic material may include an elastic film and/or elastic strands. In some laminates, a plurality of elastic strands are joined to a nonwoven while the plurality of strands are in a stretched condition so that when the elastic strands relax, the nonwoven gathers, and in turn, forms corrugations and rugosities. The resulting elastomeric laminate is stretchable to the extent that the corrugations allow the elastic strands to elongate.

Absorbent articles in the form of diaper pants may also be configured with an absorbent chassis connected with front and back elastic belts, wherein opposing end regions of the front and back belts are connected with each other at side seams. In some instances, the elasticity of the front and back belts is removed in regions where the chassis connects with the belts. Thus, in some converting configurations adapted to assemble such diaper pants, stretched elastic strands are glued between two continuous nonwoven webs to form an elastic laminate. Regions of the elastic strands may then be intermittently deactivated along the length of the elastic laminate by cutting the elastic strands in areas to be connected with the chassis, sometimes referred to as tummy elastic cutting.

Some caregivers of older incontinent babies or toddlers may prefer a closed, pant-style disposable absorbent article to enable application to, and removal from, a child while the child is in a standing position. One disadvantage of this product form is that the removal and disposal of feces-containing products may be unhygienic and inconvenient. For example, pulling the product down could cause feces to smear down the legs of a user. In other examples, a caregiver may tear open the bonded sides using force. In turn, the force used can lead to a rapid release of energy from the diaper, causing the caregiver to lose control of the product and allowing feces to spill out. In contrast, removal and disposal of traditional open or taped diaper forms with fasteners may be readily accomplished while the child is laying on their back. In this case, the fasteners are opened, the diaper is removed from under the child, rolled into a roughly cylindrical shape, and then the fasteners are secured around the rolled, soiled diaper, closing the leg openings for hygienic disposal.

In order to avoid having to remove soiled diaper pants from a wearer by sliding the soiled diaper pant down the wearer's legs or tearing bonded side seams, some diaper pants may be configured with tear lines in the front belt or back belt. Such tear lines may include perforations that allow a caregiver to more easily separate the belt along the perforation lines. Once the belt is separated, the diaper pant can be more easily removed from the wearer without having to slide the diaper pant down the wearer's legs, in a similar manner as a traditional open taped diaper form. However, due to the position of the wearer, the caregiver may not be in a position to tear along such tear lines so that the belt is severed along the tear line. Thus, the caregiver may tear the belt in an unintended manner causing the belt to tear not along the perforation line but in other locations. This can lead to more difficulty in removing the product. For example, difficulty in removing the diaper may require a caregiver to utilize both hands to start and complete the tearing operation. For example, some tear lines may require a caregiver to grasp the belt on opposing sides of the tear line and pull in opposite directions to initiate and complete the tearing process. As a result, the necessity of tearing such a belt with both hands can add difficulty to the process of removing a diaper pant from baby or child that is moving or attempting to move during the diaper removal process.

Consequently, it would be beneficial to create pant-style articles that provide the caregiver the ability to remove and dispose soiled products in a similar manner to traditional open diaper forms. **In** addition, it would be beneficial to provide diaper pants with frangible pathways configured such that the tearing operation can be initiated and completed such that the belt separates only along the frangible pathway and not in other locations of the diaper.

Pant-type absorbent articles in which a chassis is adhered to a belt and in which the belt is provided with a frangible pathway are disclosed in US 8192417 B2 and US 8043274 B2.

### SUMMARY

The invention provides an absorbent article according to claim 1. Optional features of the invention are set out in the dependent claims.

In one form, an absorbent article comprises: a chassis comprising a topsheet, a backsheet, and an absorbent core positioned between the topsheet and the backsheet, the chassis comprising a longitudinally extending first side edge and a longitudinally extending second side edge laterally separated from the first side edge by a first end edge and a second end edge longitudinally separate from the first end edge; a first belt joined to a portion of the chassis, wherein the first belt comprises a first substrate, wherein the first substrate comprises a first frangible pathway, wherein at least a portion of the first frangible pathway extends at a pathway angle of less than 90 degrees from a lateral centerline of the first belt, wherein the first substrate has a material tear strength according to the Trap-Tear Method and a material basis weight, wherein the first frangible pathway has a pathway tear strength according to the Trap-Tear Method, wherein the material tear strength of the first substrate is greater than the pathway tear strength of the first frangible pathway, and wherein the material tear strength of the first substrate is at least 2 N greater than the pathway tear strength of the first frangible pathway.

In another form, an absorbent article comprises: a chassis comprising a topsheet, a backsheet, and an absorbent core positioned between the topsheet and the backsheet, the chassis comprising a longitudinally extending first side edge and a longitudinally extending second side edge laterally separated from the first side edge by a first end edge and a second end edge longitudinally separate from the first end edge; a first belt joined to a portion of the chassis and having a longitudinal centerline and a lateral centerline substantially perpendicular to the longitudinal centerline, wherein the first belt comprises a frangible pathway, wherein at least a portion of the frangible pathway extends at a pathway angle of less than 90 degrees from the lateral centerline of the first belt, wherein the first belt has a material tear strength according to the Trap-Tear Method and a material basis weight, wherein the frangible pathway has a pathway tear strength according to the Trap-Tear Method, wherein the material tear strength of the first belt is at least 2 N greater than the pathway tear strength of the first frangible pathway.

In yet another form, an absorbent article comprises: a chassis comprising a topsheet, a backsheet, and an absorbent core positioned between the topsheet and the backsheet, the chassis comprising a longitudinally extending first side edge and a longitudinally extending second side edge laterally separated from the first side edge by a first end edge and a second end edge longitudinally separate from the first end edge; a first belt comprising an inner wearer facing surface and an outer garment facing surface, the first belt further comprising a laterally extending inner edge and a laterally extending outer edge, the outer edge positioned longitudinally outward of the inner edge; a frangible pathway in the first belt extending between the inner edge and the outer edge of the first belt, wherein the first belt has a material tear strength according to the Trap-Tear Method and a first belt basis weight, wherein the frangible pathway has a pathway tear strength according to the Trap-Tear Method, wherein the tear strength of the first belt divided by the substrate basis weight is greater than 0.2 N/gsm, wherein the tear strength of the frangible pathway divided by the first basis weight is greater than 0.05 N/gsm and less than 0.15 N/gsm, and wherein the difference between the material tear strength and the pathway tear strength is a tear strength delta, and wherein the tear strength delta divided by the material basis weight is greater than 0.2 N/gsm.

In yet another form, an absorbent article comprises: a chassis comprising a topsheet, a backsheet, and an absorbent core positioned between the topsheet and the backsheet, the chassis comprising a longitudinally extending first side edge and a longitudinally extending second side edge laterally separated from the first side edge by a first end edge and a second end edge longitudinally separate from the first end edge; a first belt joined to a portion of the chassis and having a longitudinal centerline and a lateral centerline substantially perpendicular to the longitudinal centerline, wherein the first belt comprises a first substrate and a frangible pathway, wherein the first substrate of the first belt comprises a nonwoven having a majority of the fibers oriented in a fiber orientation direction, wherein the frangible pathway has a pathway angle, wherein the pathway angle of at least a portion of the frangible pathway is less than 90 degrees from the fiber orientation direction, wherein the first substrate has a tear strength according to the Trap-Tear Method and a substrate basis weight, wherein the frangible pathway has a tear strength according to the Trap-Tear Method, and wherein the tear strength of the first substrate is greater than the tear strength of the frangible pathway.

### BRIEF DESCRIPTION OF THE DRAWINGS

Figure 1 shows a perspective view of a diaper pant in a pre-fastened configuration.
Figure 1A shows a perspective view of a diaper pant with a continuous outer cover in a pre-fastened configuration.
Figure 2A shows a plan view of a diaper pant with the portion of the diaper that faces away from a wearer oriented toward the viewer.
Figure 2B shows a plan view of a diaper pant with the portion of the diaper that faces toward a wearer oriented toward the viewer.
Figure 2C shows a plan view of a diaper pant with the portion of the diaper that faces away from a wearer oriented toward the viewer, illustrating first and second belt size and shape features.
Figure 2D shows a plan view of a diaper pant with the portion of the diaper that faces away from a wearer oriented toward the viewer, illustrating first and second belt size and shape features.
Figure 2E shows a plan view of a diaper pant with the portion of the diaper that faces away from a wearer oriented toward the viewer, illustrating first and second belt size and shape features.
Figure 2F shows a plan view of a diaper pant with a continuous outer cover with the portion of the diaper that faces away from a wearer oriented toward the viewer.
Figure 3 is a cross-sectional view of the diaper pant of Figure 2A taken along line 3-3 showing first and second elastic belts provided with panel layers.
Figure 3A is a cross-sectional detailed view of a first belt provided with panel layers wherein one panel layer is folded over another panel layer.

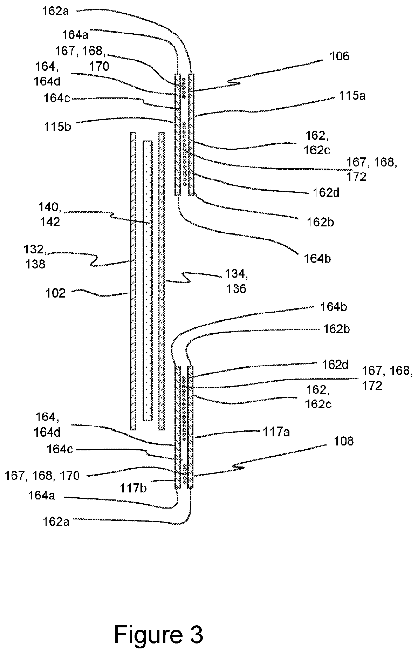

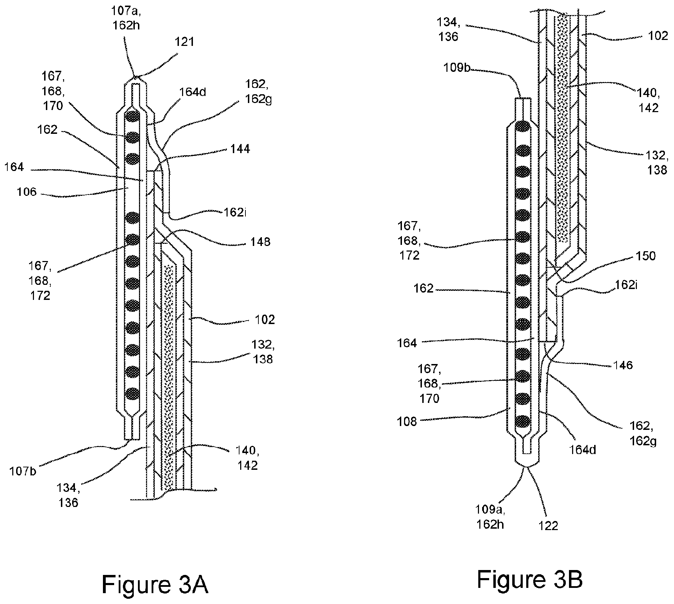

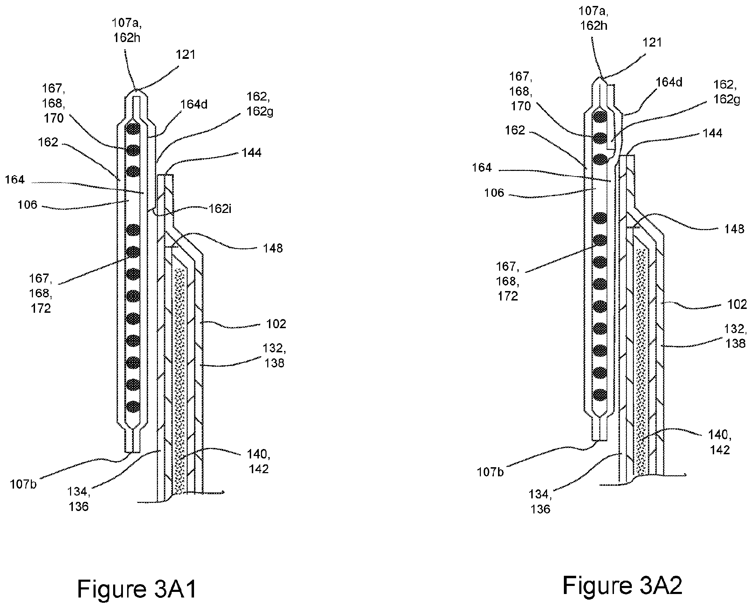

Figure 3A1 is a cross-sectional detailed view of another example configuration wherein the first belt is provided with panel layers wherein one panel layer is folded over another panel layer.
Figure 3A2 is a cross-sectional detailed view of another example configuration wherein the first belt is provided with panel layers wherein one panel layer is folded over another panel layer.
Figure 3B is a cross-sectional detailed view of a second belt provided with panel layers wherein one panel layer is folded over another panel layer.

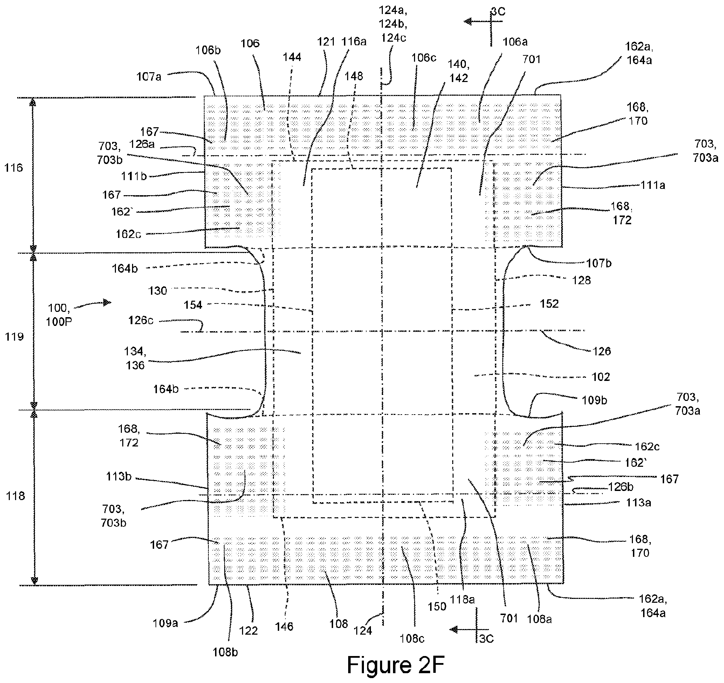

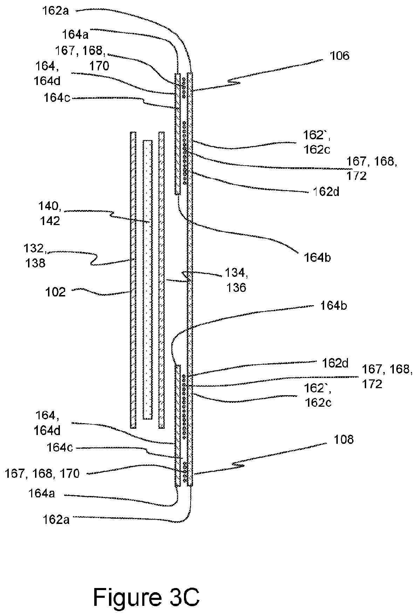

Figure 3C is a cross-sectional view of the diaper pant of Figure 2F taken along line 3C-3C showing first and second elastic belts provided with panel layers and a continuous outer cover.

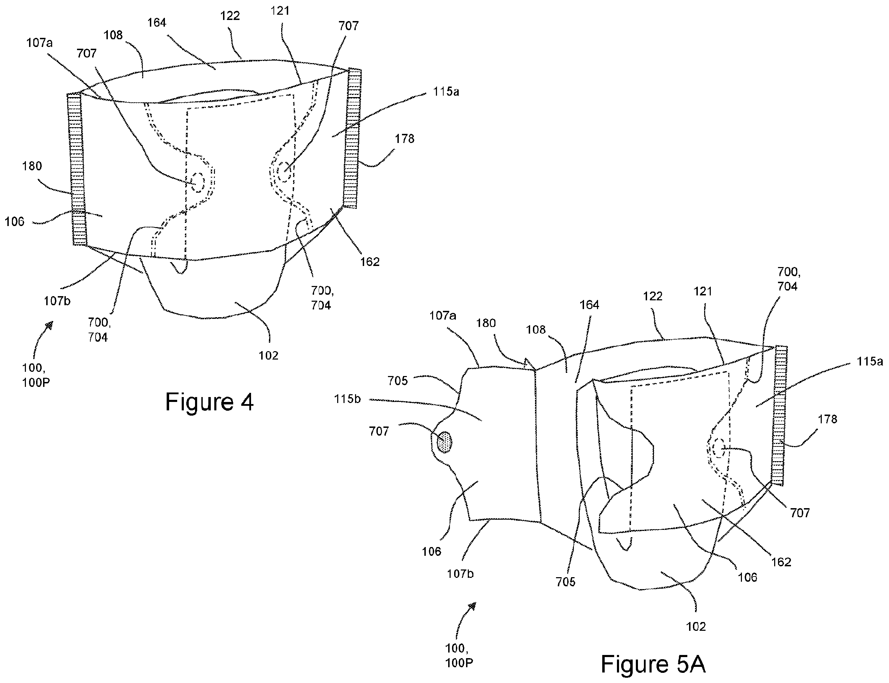

Figure 4 is perspective view of a diaper pant including frangible pathways in a front belt and adjacent an absorbent chassis.
Figure 5A is a perspective view of the diaper pant of Figure 4 showing the front belt having been torn along one of the frangible pathways.

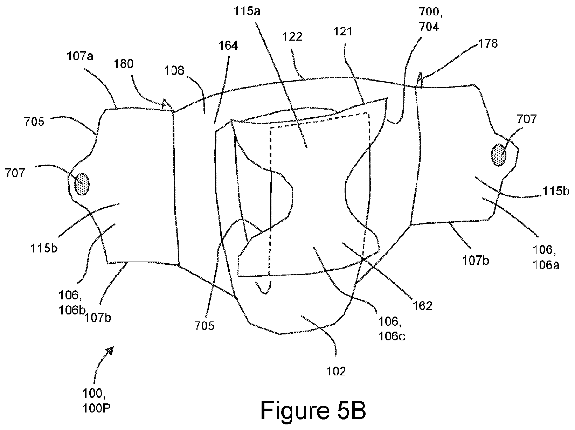

Figure 5B is a perspective view of the diaper pant of Figure 4 showing the front belt having been torn along two frangible pathways.

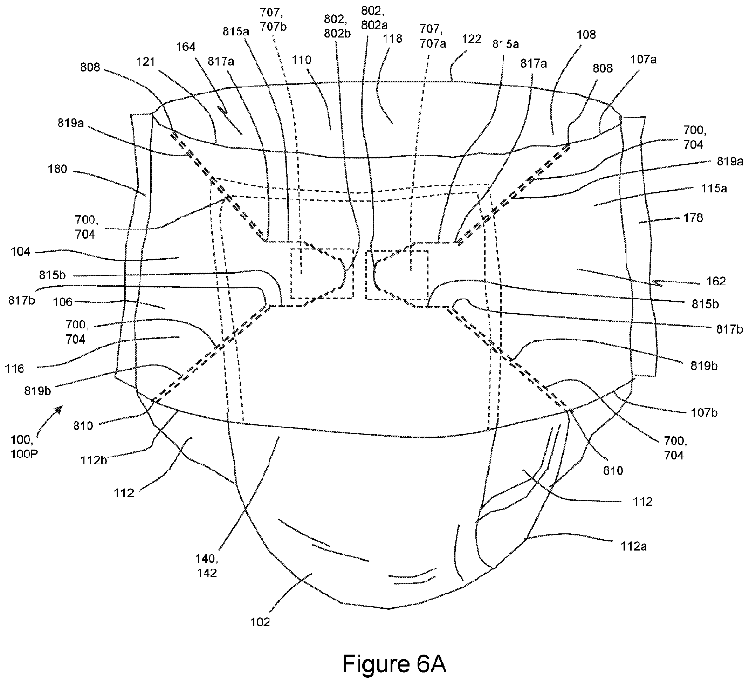

Figure 6A is a perspective view of a diaper pant with frangible pathways.

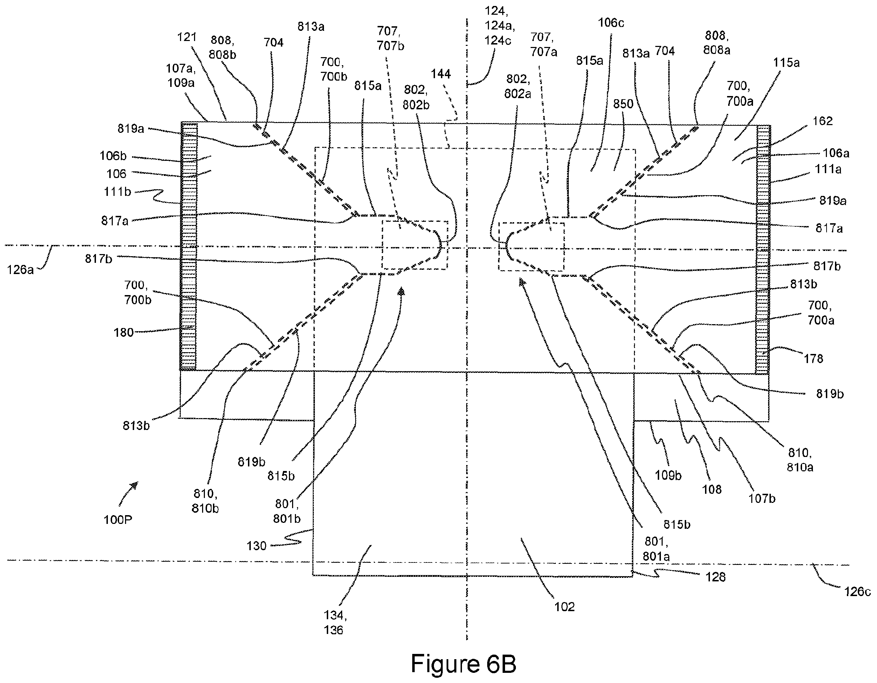

Figure 6B is a front plan view of the diaper pant of Figure 6A.

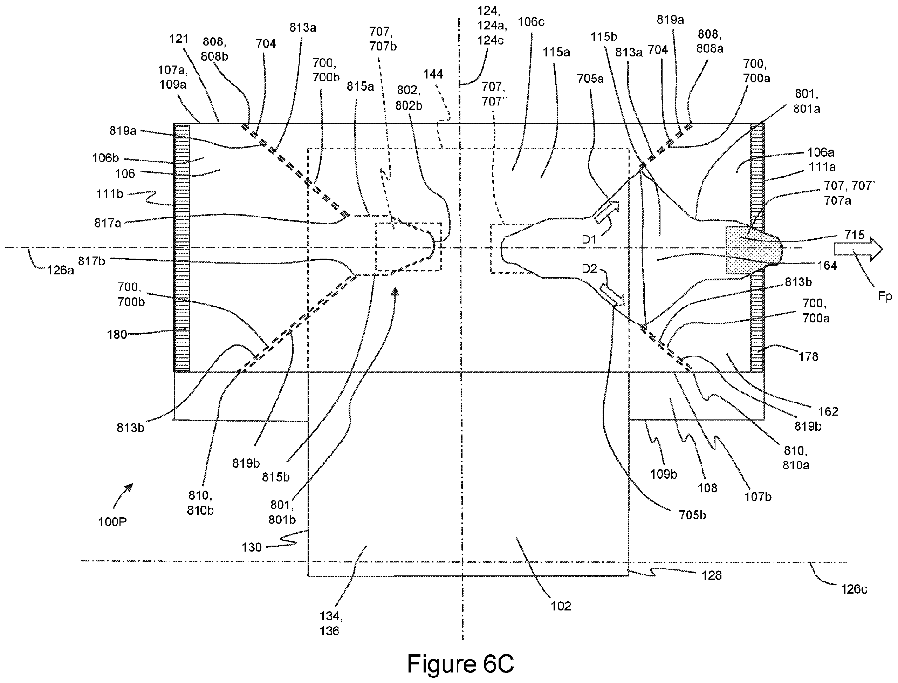

Figure 6C shows a front plan view of the diaper pant of Figure 6B as a first frangible pathway is being torn.

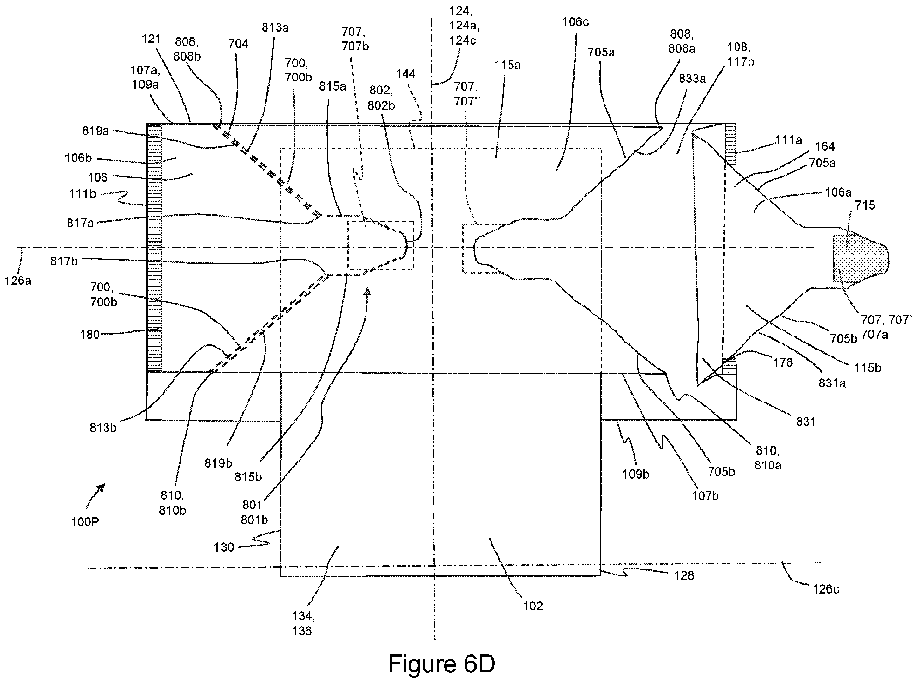

Figure 6D shows a front plan view of the diaper pant of Figure 6C after the first frangible pathway has been completely torn.

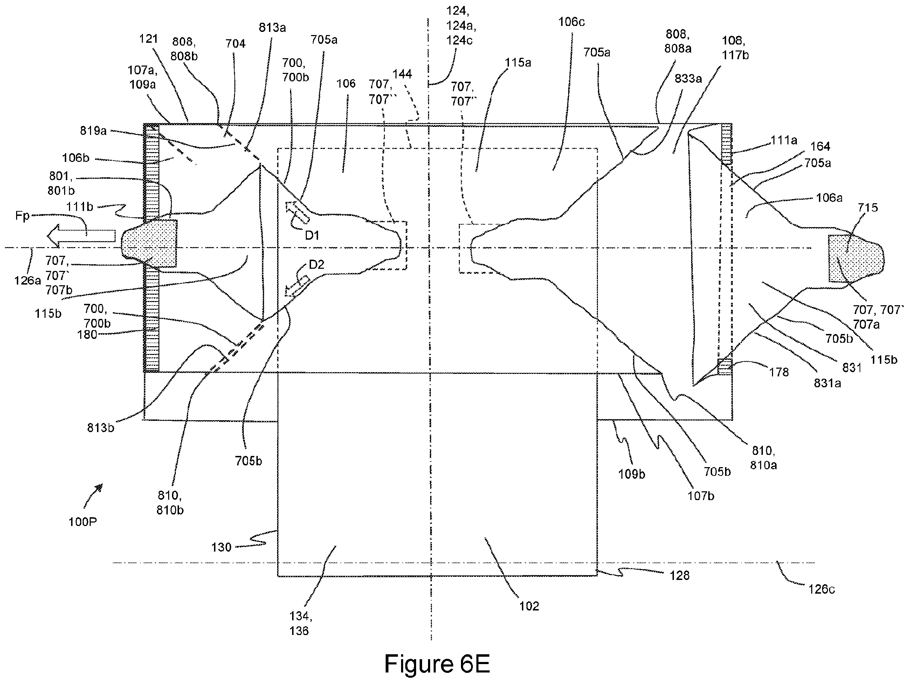

Figure 6E shows a front plan view of the diaper pant of Figure 6D as a second frangible pathway is being torn.

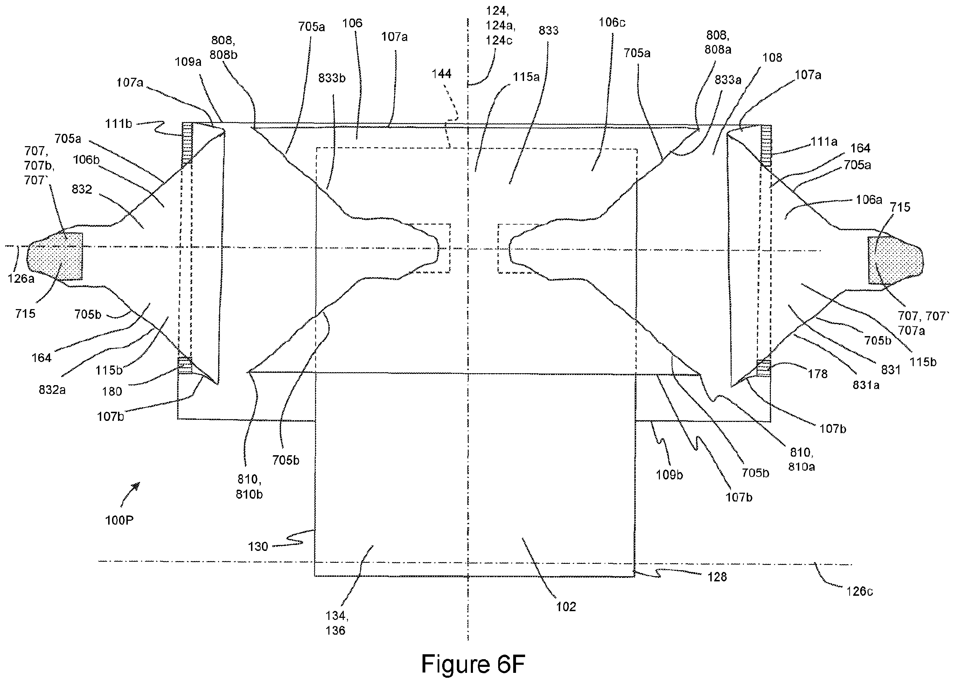

Figure 6F shows a front plan view of the diaper pant of Figure 6E after the second frangible pathway has been completely torn.

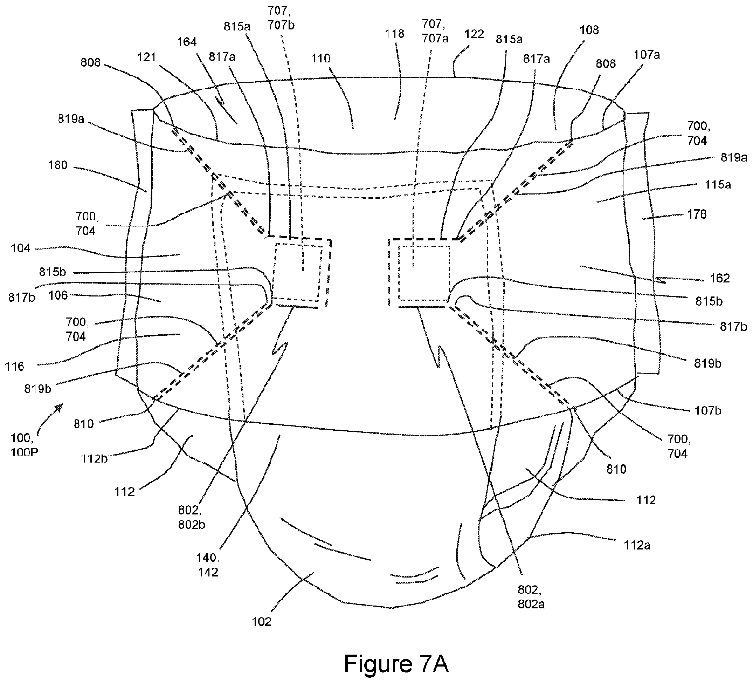

Figure 7A is a perspective view of a diaper pant with another configuration of frangible pathways.

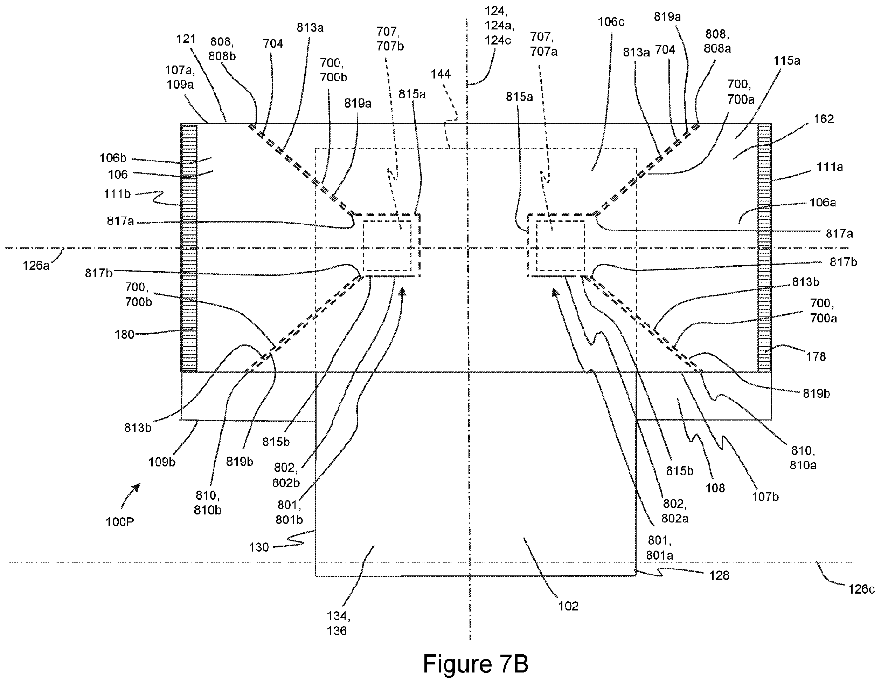

Figure 7B is a front plan view of the diaper pant of Figure 7A.

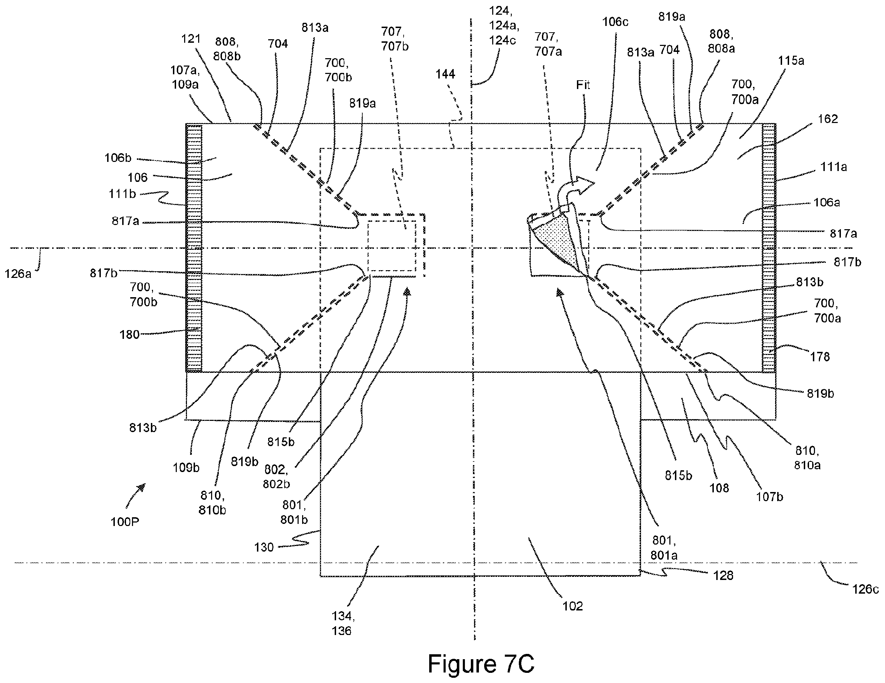

Figure 7C shows a front plan view of the diaper pant of Figure 7B as a first frangible pathway is being torn along first and second initial tear zones.

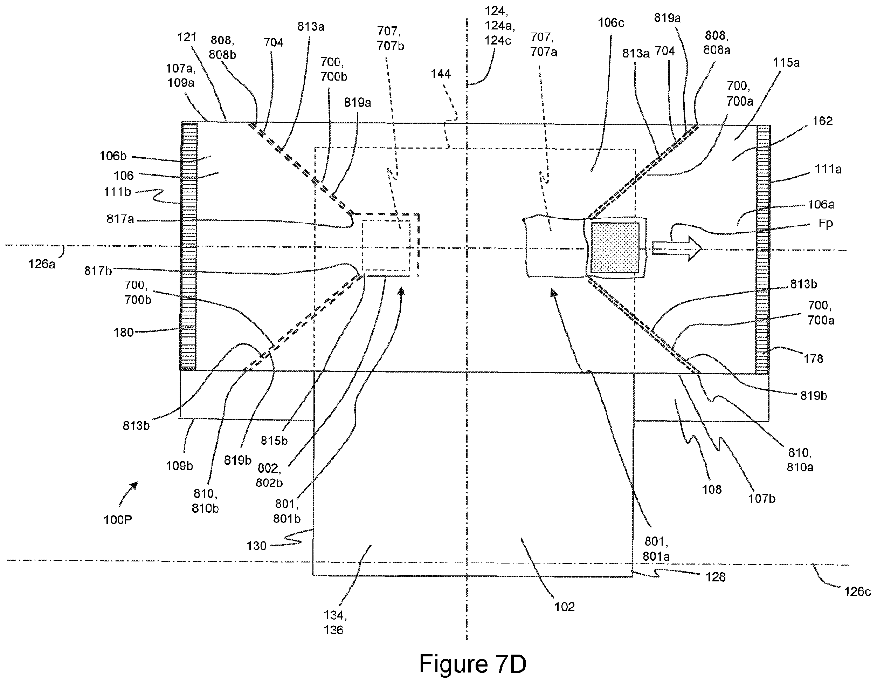

Figure 7D shows a front plan view of the diaper pant of Figure 7C after a first frangible pathway has been torn along first and second initial tear zones.

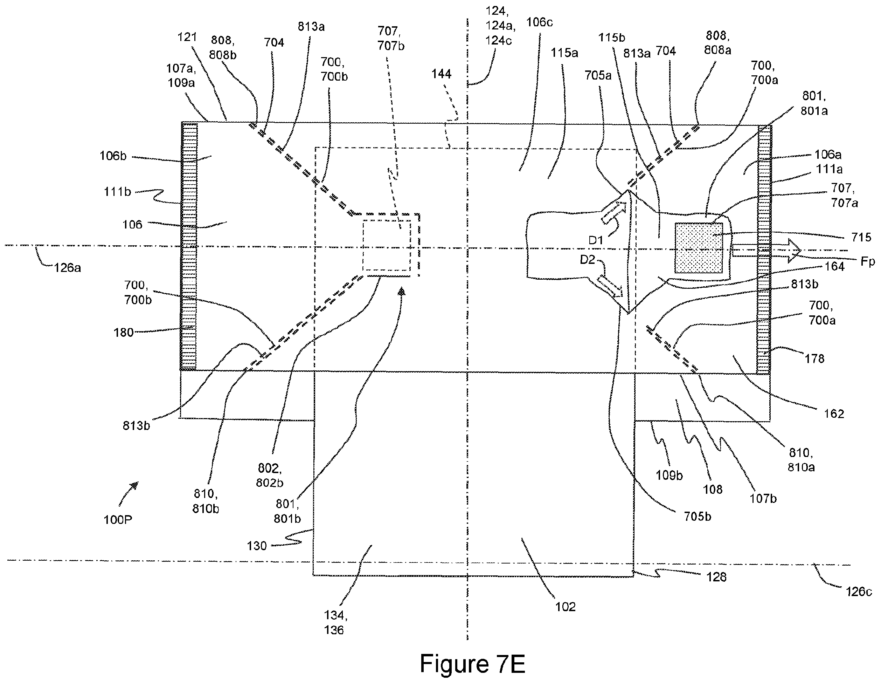

Figure 7E shows a front plan view of the diaper pant of Figure 7D as the first frangible pathway is being torn along first and second secondary tear zones.

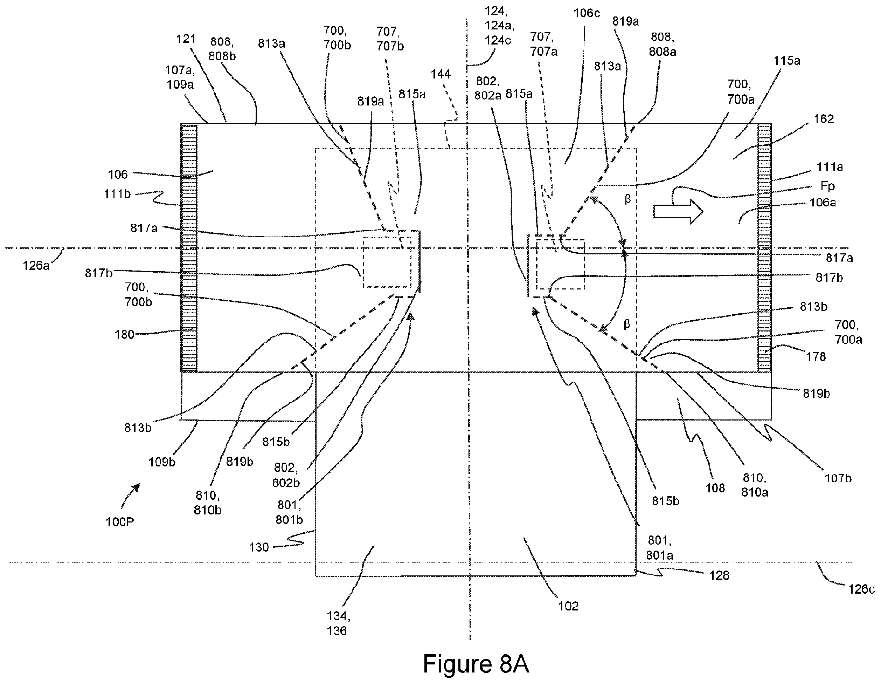

Figure 8A is a front plan view of a diaper pant with another configuration of frangible pathways.

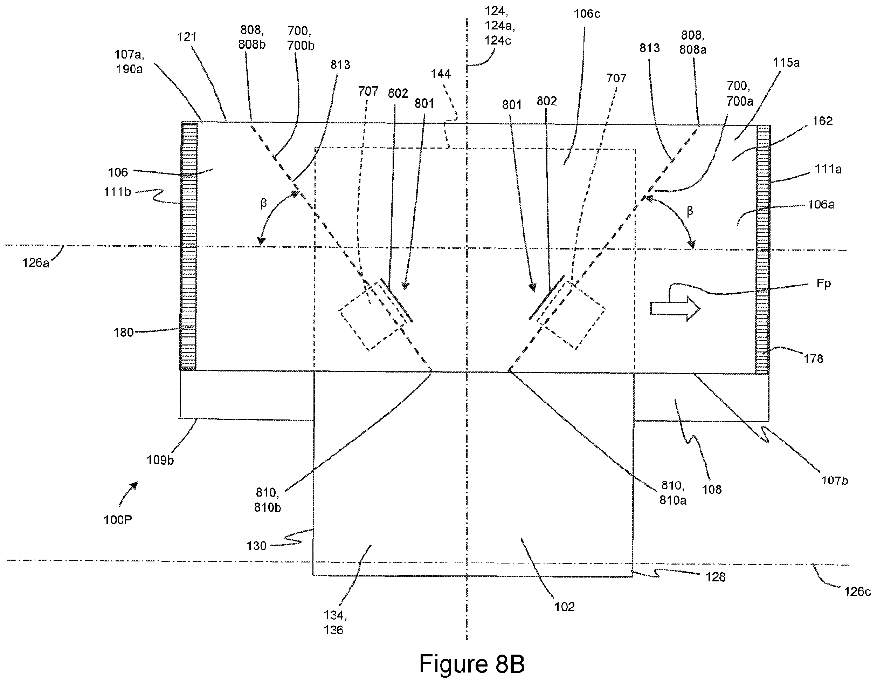

Figure 8B is a front plan view of a diaper pant with another configuration of frangible pathways.

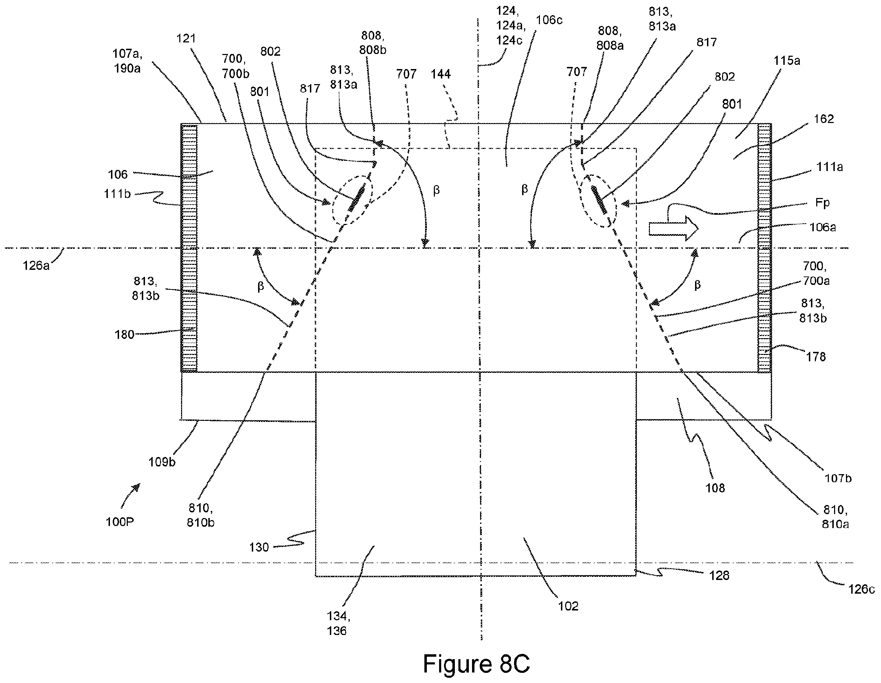

Figure 8C is a front plan view of a diaper pant with another configuration of frangible pathways.

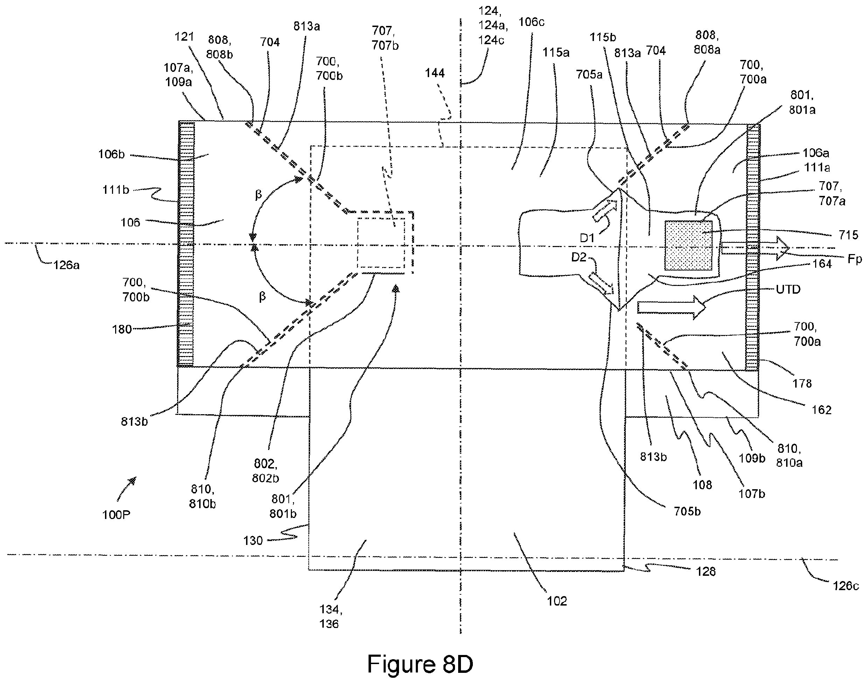

Figure 8D shows a front plan view of the diaper pant of Figure 8A as the first frangible pathway is being torn along first and second secondary tear zones.

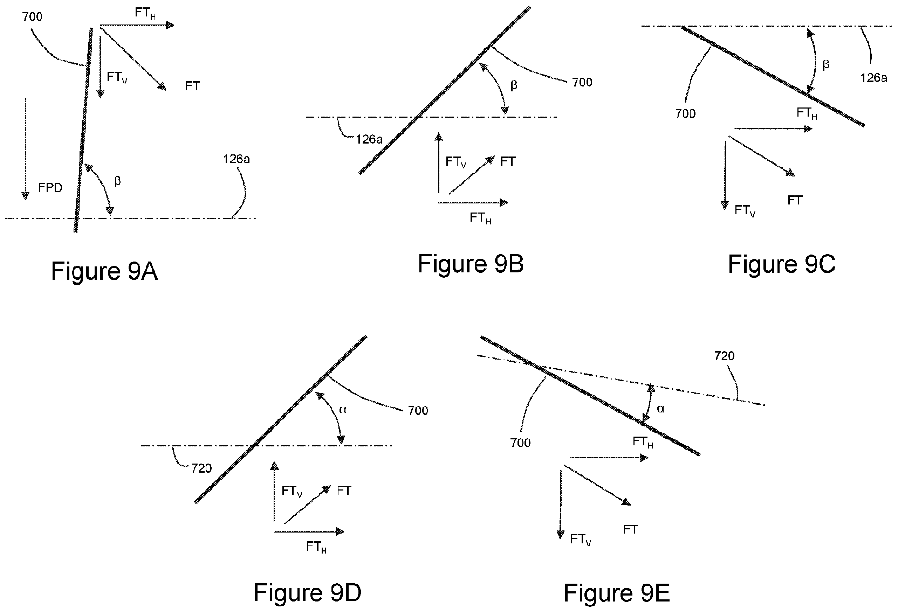

Figure 9A is a schematic illustration of a frangible pathway direction in relation to the force to tear direction and the pathway angle with respect to the lateral centerline.
Figure 9B is a schematic illustration of a frangible pathway direction in relation to the force to tear direction and the pathway angle with respect to the lateral centerline.
Figure 9C is a schematic illustration of a frangible pathway direction in relation to the force to tear direction and the pathway angle with respect to the lateral centerline.
Figure 9D is a schematic illustration of a frangible pathway direction in relation to the force to tear direction and the pathway angle with respect to the fiber orientation direction.
Figure 9E is a schematic illustration of a frangible pathway direction in relation to the force to tear direction and the pathway angle with respect to the fiber orientation direction.

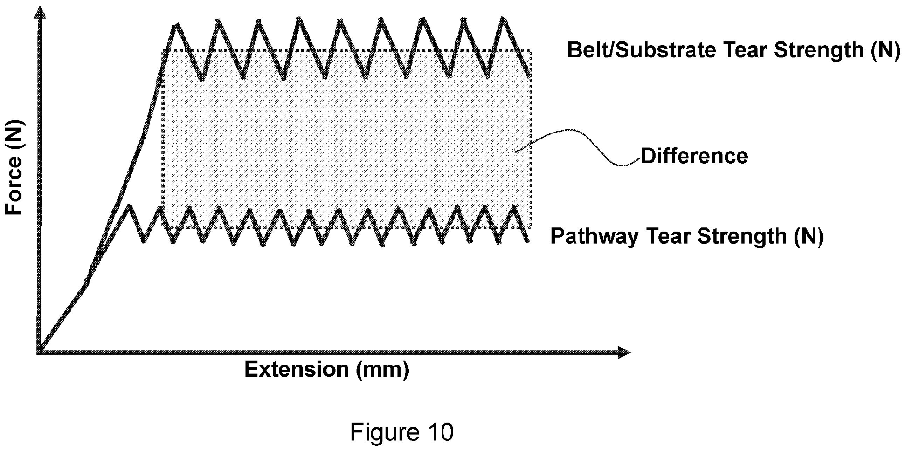

Figure 10 is a schematic illustration of the difference between the belt tear strength and the pathway tear strength.

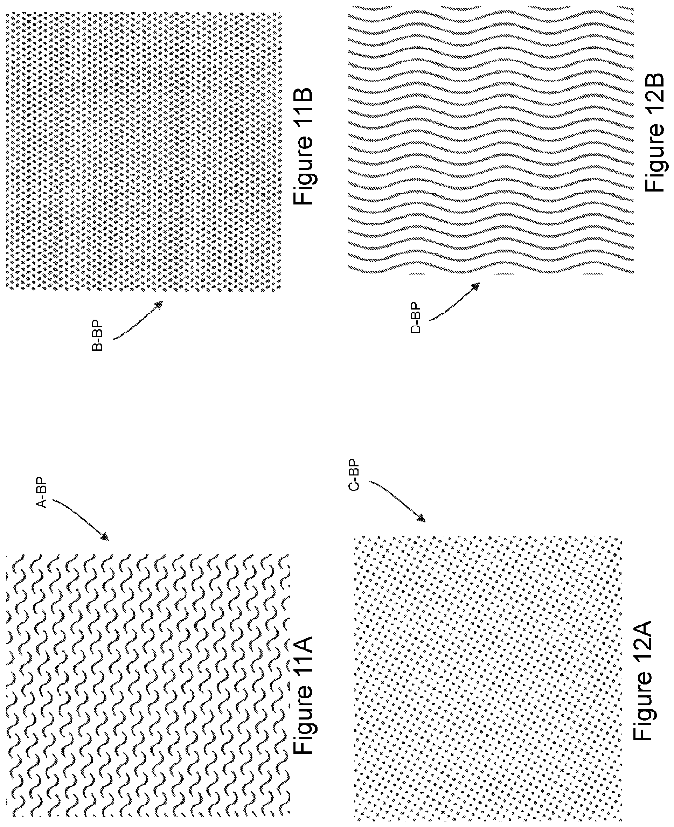

Figure 11A is a schematic illustration of a nonwoven bond pattern.
Figure 11B is a schematic illustration of a nonwoven bond pattern.
Figure 12A is a schematic illustration of a nonwoven bond pattern.
Figure 12B is a schematic illustration of a nonwoven bond pattern.

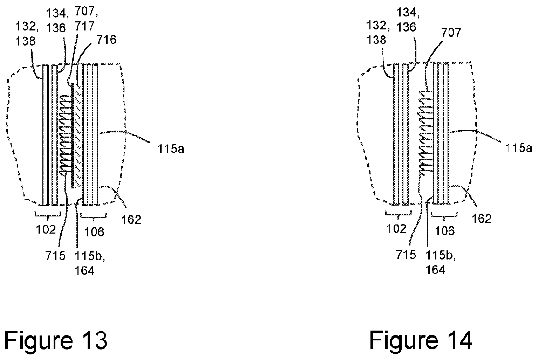

Figure 13 is a cross-sectional view of the fastener component of Figure 8A.
Figure 14 is a cross-sectional view of the fastener component of Figure 8A wherein the fastener component is integrally formed from belt components.

### DETAILED DESCRIPTION

The following term explanations may be useful in understanding the present disclosure:
"Absorbent article" refers to devices, which absorb and contain body exudates and, more specifically, refers to devices, which are placed against or in proximity to the body of the wearer to absorb and contain the various exudates discharged from the body. Exemplary absorbent articles include diapers, training pants, pull-on pant-type diapers (i.e., a diaper having a pre-formed waist opening and leg openings such as illustrated in U.S. Pat. No. 6,120,487), refastenable diapers or pant-type diapers, incontinence briefs and undergarments, diaper holders and liners, feminine hygiene garments such as panty liners, absorbent inserts, menstrual pads and the like.
"Body-facing" and "garment-facing" refer respectively to the relative location of an element or a surface of an element or group of elements. "Body-facing" implies the element or surface is nearer to the wearer during wear than some other element or surface. "Garment-facing" implies the element or surface is more remote from the wearer during wear than some other element or surface (i.e., element or surface is proximate to the wearer's garments that may be worn over the disposable absorbent article).

The terms "elastic," "elastomer" or "elastomeric" refers to materials exhibiting elastic properties, which include any material that upon application of a force to its relaxed, initial length can stretch or elongate to an elongated length more than 10% greater than its initial length and will substantially recover back to about its initial length upon release of the applied force. Elastomeric materials may include elastomeric films, scrims, nonwovens, ribbons, strands and other sheet-like structures.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

As used herein, the term "distal" is used to describe a position situated away from a center of a body or from a point of attachment, and the term "proximal" is used to describe a position situated nearer to a center of a body or a point of attachment.

The term "substrate" is used herein to describe a material which is primarily two-dimensional (i.e., in an XY plane) and whose thickness (in a Z direction) is relatively small (i.e., 1/10 or less) in comparison to its length (in an X direction) and width (in a Y direction). Nonlimiting examples of substrates include a web, layer or layers or fibrous materials, nonwovens, films and foils such as polymeric films or metallic foils. These materials may be used alone or may comprise two or more layers laminated together. As such, a web is a substrate.

The term "nonwoven" refers herein to a material made from continuous (long) filaments (fibers) and/or discontinuous (short) filaments (fibers) by processes such as spunbonding, meltblowing, carding, and the like. Nonwovens do not have a woven or knitted filament pattern.

The term "machine direction" (MD) is used herein to refer to the direction of material flow through a process. In addition, relative placement and movement of material can be described as flowing in the machine direction through a process from upstream in the process to downstream in the process.

The term "cross direction" (CD) is used herein to refer to a direction that is generally perpendicular to the machine direction.

"Pre-strain" refers to the strain imposed on an elastic or elastomeric material prior to combining it with another element of the elastomeric laminate or the absorbent article. Pre-strain is determined by the following equation Pre-strain = ((extended length of the elastic-relaxed length of the elastic)/relaxed length of the elastic)*100.

"Decitex" also known as Dtex is a measurement used in the textile industry used for measuring yarns or filaments. 1 Decitex = 1 gram per 10,000 meters. In other words, if 10,000 linear meters of a yarn or filament weights 500 grams that yarn or filament would have a decitex of 500.

The term "taped diaper" (also referred to as "open diaper") refers to disposable absorbent articles having an initial front waist region and an initial back waist region that are not fastened, pre-fastened, or connected to each other as packaged, prior to being applied to the wearer. A taped diaper may be folded about the lateral centerline with the interior of one waist region in surface to surface contact with the interior of the opposing waist region without fastening or joining the waist regions together. Example taped diapers are disclosed in various suitable configurations U.S. Patent Nos. 5,167,897, 5,360,420, 5,599,335, 5,643,588, 5,674,216, 5,702,551, 5,968,025, 6,107,537, 6,118,041, 6,153,209, 6,410,129, 6,426,444, 6,586,652, 6,627,787, 6,617,016, 6,825,393, and 6,861,571; and U.S. Patent Publication Nos. 2013/0072887 A1; 2013/0211356 A1; and 2013/0306226 A1,

The term "pant" (also referred to as "training pant", "pre-closed diaper", "diaper pant", "pant diaper", and "pull-on diaper") refers herein to disposable absorbent articles having a continuous perimeter waist opening and continuous perimeter leg openings designed for infant or adult wearers. A pant can be configured with a continuous or closed waist opening and at least one continuous, closed, leg opening prior to the article being applied to the wearer. A pant can be preformed or pre-fastened by various techniques including, but not limited to, joining together portions of the article using any refastenable and/or permanent closure member (e.g., seams, heat bonds, pressure welds, adhesives, cohesive bonds, mechanical fasteners, etc.). A pant can be preformed anywhere along the circumference of the article in the waist region (e.g., side fastened or seamed, front waist fastened or seamed, back waist fastened or seamed). Example diaper pants in various configurations are disclosed in U.S. Patent Nos. 4,940,464; 5,092,861; 5,246,433; 5,569,234; 5,897,545; 5,957,908; 6,120,487; 6,120,489; 7,569,039 and U.S. Patent Publication Nos. 2003/0233082 A1; 2005/0107764 A1, 2012/0061016 A1, 2012/0061015 A1; 2013/0255861 A1; 2013/0255862 A1; 2013/0255863 A1; 2013/0255864 A1; and 2013/0255865 A1,

"Closed-form" means opposing waist regions are joined, as packaged, either permanently or refastenably to form a continuous waist opening and leg openings.

"Open-form" means opposing waist regions are not initially joined to form a continuous waist opening and leg openings but comprise a closure means such as a fastening system to join the waist regions to form the waist and leg openings before or during application to a wearer of the article.

The present disclosure relates to absorbent articles including elastic laminates, and more particularly, to absorbent articles having elastic laminates in front and/or back waist regions with one or more frangible pathways. In some configurations, an absorbent article may comprise: a first belt and a second belt, each belt comprising a first end region and a second end region laterally separated from the first end region by a central region. The first end region of the first belt is connected with the first end region of the second belt and the second end region of the first belt is connected with the second end region of the second belt to form a waist opening. The absorbent article may further comprise a chassis comprising a topsheet, a backsheet, and an absorbent core positioned between the topsheet and the backsheet. The chassis may further comprise a first end region and a second end region longitudinally separated from the first end region by a crotch region. The first end region of the chassis may be connected with the central region of the first belt and the second end region of the chassis may be connected with the central region of the second belt. The first belt may further comprise a laterally extending inner edge and a laterally extending outer edge, the outer edge positioned longitudinally outward of the inner edge.

As discussed in more detail below, the first belt and/or the second belt may comprise one or more frangible pathways. For example, a frangible pathway in the first and/or second belt may extend between the inner edge of the first belt and the outer edge of the first belt. The frangible pathway may extend to a proximal terminus on the inner edge and a distal terminus on the outer edge of the first belt. In turn, the frangible pathway may comprise a single tear zone or a first tear zone and a second tear zone. Such frangible pathway configurations provide a feature that allows an elastic belt of a diaper pant to be relatively easily torn along the frangible pathway when removing the diaper pant from a wearer, avoiding the need to remove the diaper pant by sliding the diaper pant over a wearer's legs. The tear strength needed to tear along the frangible pathway may be significantly less than the tear strength to tear the belt so that the tear only propagates along the frangible pathway and not into other areas of the belt. In addition, the frangible pathway may be configured to allow a caregiver or wearer to initiate and/or completely tear the belt with one hand when removing a diaper pant from a wearer.

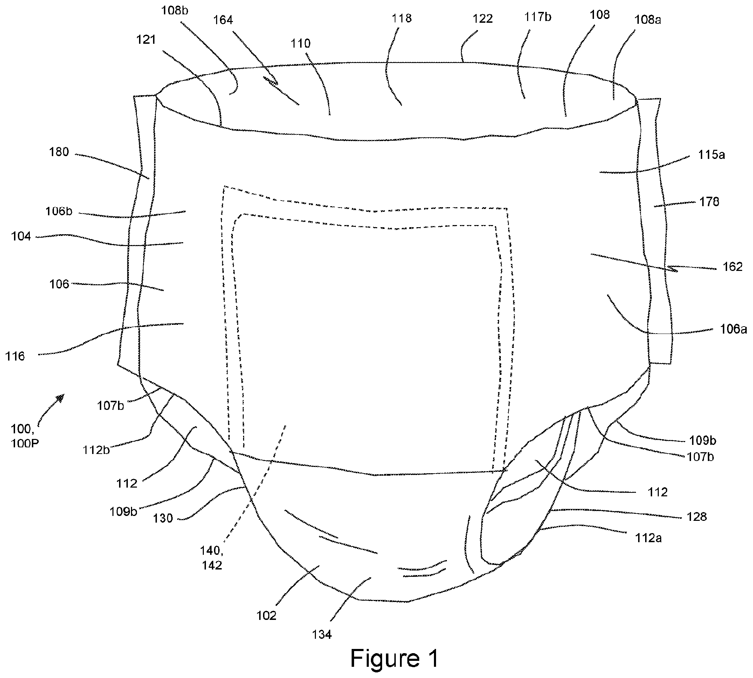

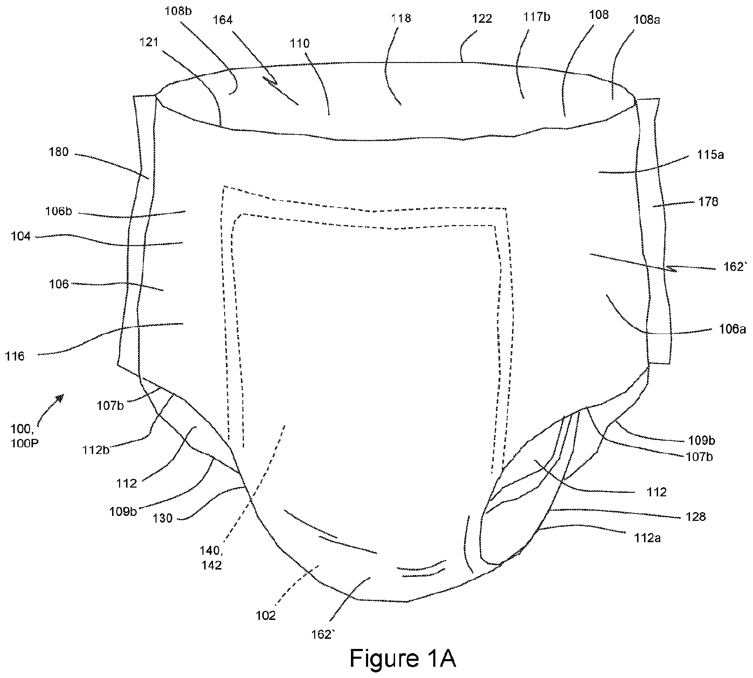

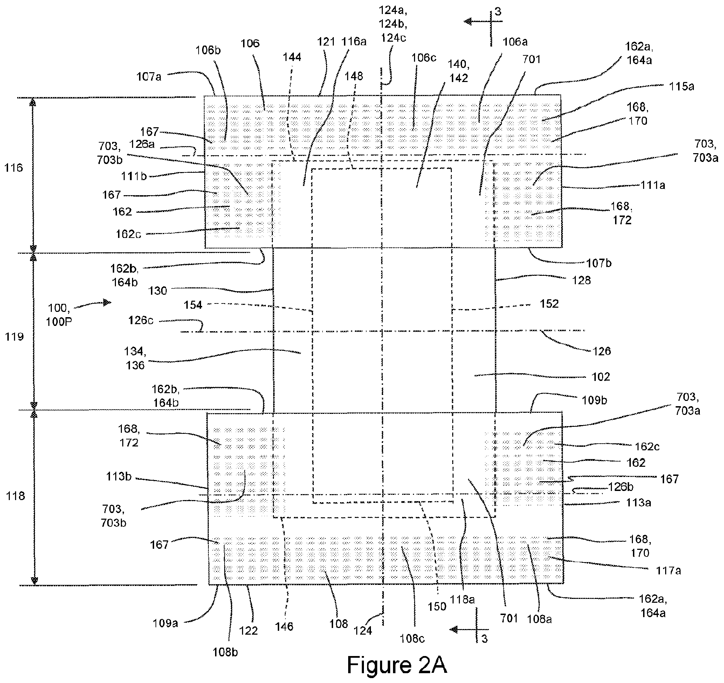

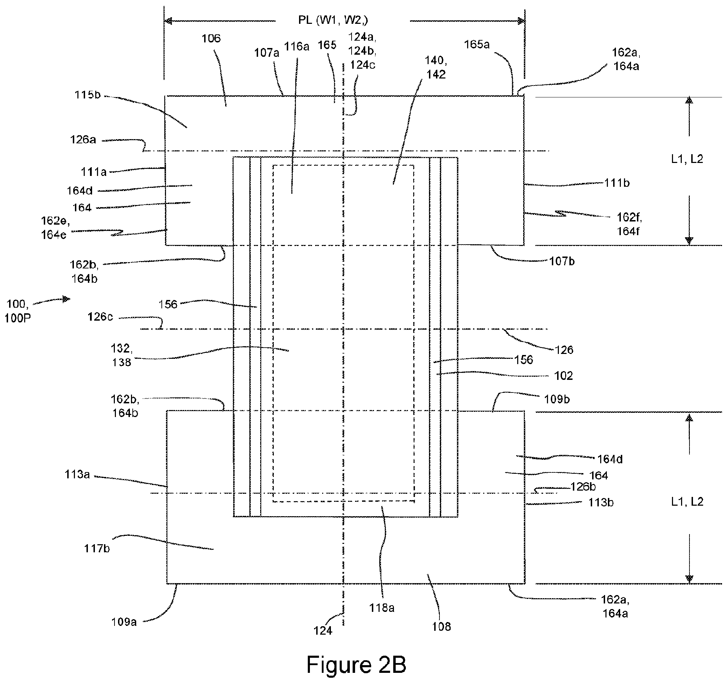

Figures 1-2B show an example of an absorbent article 100 in the form of a diaper pant 100P that may include components constructed in accordance with the configurations disclosed herein. In particular, Figure 1 shows a perspective views of a diaper pant 100P in a pre-fastened configuration. Figure 2A shows a plan view of the diaper pant 100P with the portion of the diaper that faces away from a wearer oriented toward the viewer, and Figure 2B shows a plan view of the diaper pant 100P with the portion of the diaper that faces toward a wearer oriented toward the viewer. The diaper pant 100P includes a chassis 102 and a ring-like elastic belt 104. As discussed below in more detail, a first elastic belt 106 and a second elastic belt 108 are bonded together to form the ring-like elastic belt 104.

With continued reference to Figures 1-2B, the diaper pant 100P and the chassis 102 each include a first waist region 116, a second waist region 118, and a crotch region 119 disposed intermediate the first and second waist regions. It may also be described that the chassis 102 includes a first end region 116a, a second end region 118a, and a crotch region 119 disposed intermediate the first and second end regions 116a, 118a. The first waist region 116 may be configured as a front waist region, and the second waist region 118 may be configured as back waist region. The diaper 100P may also include a laterally extending front waist edge 121 in the front waist region 116 and a longitudinally opposing and laterally extending back waist edge 122 in the back waist region 118. To provide a frame of reference for the present discussion, the diaper 100P and chassis 102 of Figures 2A and 2B are shown with a longitudinal axis 124 and a lateral axis 126. In some embodiments, the longitudinal axis 124 may extend through the front waist edge 121 and through the back waist edge 122. And the lateral axis 126 may extend through a first longitudinal or right side edge 128 and through a second longitudinal or left side edge 130 of the chassis 102. As previously mentioned, the longitudinal axis 124 extends perpendicularly through the front waist edge 121 and the back waist edge 122, and the lateral axis 126 extends perpendicularly to the longitudinal axis 124. When the diaper pant 100P is worn, the longitudinal direction may extend from the wearer's front waist, through the crotch, to the wearer's back waist. To provide a further frame of reference for the present discussion, the diapers 100P of Figures 2A, 2B, and 18B are shown wherein: the first elastic belt 106 comprises a longitudinal centerline 124a and lateral centerline 126a; the second elastic belt 108 comprises a longitudinal centerline 124b and lateral centerline 126b; and the chassis 102 comprises a longitudinal centerline 124c and lateral centerline 126c. The longitudinal centerlines 124a, 124b, 124c are perpendicular to the lateral center lines 126a, 126b, 126c.

As shown in Figures 1-2B, the diaper pant 100P may include an inner, body facing surface 132, and an outer, garment facing surface 134. The chassis 102 may include a backsheet 136 and a topsheet 138. The chassis 102 may also include an absorbent assembly 140, including an absorbent core 142, disposed between a portion of the topsheet 138 and the backsheet 136. As discussed in more detail below, the diaper 100P may also include other features, such as leg elastics and/or leg cuffs to enhance the fit around the legs of the wearer.

As shown in Figure 2A, the periphery of the chassis 102 may be defined by the first longitudinal side edge 128, a second longitudinal side edge 130, a first laterally extending end edge 144 disposed in the first waist region 116, and a second laterally extending end edge 146 disposed in the second waist region 118. Both side edges 128 and 130 extend longitudinally between the first end edge 144 and the second end edge 146. As shown in Figure 2A, the laterally extending end edges 144 and 146 may be located longitudinally inward from the laterally extending front waist edge 121 in the front waist region 116 and the laterally extending back waist edge 122 in the back waist region 118. In some configurations, the laterally extending end edges 144 and 146 may be coterminous with or located longitudinally outward from the laterally extending front waist edge 121 in the front waist region 116 and the laterally extending back waist edge 122 in the back waist region 118. When the diaper pant 100P is worn on the lower torso of a wearer, the front waist edge 121 and the back waist edge 122 may encircle a portion of the waist of the wearer. At the same time, the side edges 128 and 130 may encircle at least a portion of the legs of the wearer. And the crotch region 119 may be generally positioned between the legs of the wearer with the absorbent core 142 extending from the front waist region 116 through the crotch region 119 to the back waist region 118.

As previously mentioned, the diaper pant 100P may include a backsheet 136. The backsheet 136 may also define the outer, garment facing surface 134 of the chassis 102. The backsheet 136 may also comprise a woven or nonwoven material, polymeric films such as thermoplastic films of polyethylene or polypropylene, and/or a multi-layer or composite materials comprising a film and a nonwoven material. The backsheet may also comprise an elastomeric film. An example backsheet 136 may be a polyethylene film having a thickness of from about 0.012 mm (0.5 mils) to about 0.051 mm (2.0 mils). Further, the backsheet 136 may permit vapors to escape from the absorbent core (i.e., the backsheet is breathable) while still preventing exudates from passing through the backsheet 136.

Also described above, the diaper pant 100P may include a topsheet 138. The topsheet 138 may also define all or part of the inner, wearer facing surface 132 of the chassis 102. The topsheet 138 may be liquid pervious, permitting liquids (e.g., menses, urine, and/or runny feces) to penetrate through its thickness. A topsheet 138 may be manufactured from a wide range of materials such as woven and nonwoven materials; apertured or hydroformed thermoplastic films; apertured nonwovens, porous foams; reticulated foams; reticulated thermoplastic films; and thermoplastic scrims. Woven and nonwoven materials may comprise natural fibers such as wood or cotton fibers; synthetic fibers such as polyester, polypropylene, or polyethylene fibers; or combinations thereof. If the topsheet 138 includes fibers, the fibers may be spunbond, carded, wet-laid, meltblown, hydroentangled, or otherwise processed as is known in the art. Topsheets 138 may be selected from high loft nonwoven topsheets, apertured film topsheets and apertured nonwoven topsheets. Exemplary apertured films may include those described in U.S. Patent Nos. 5,628,097; 5,916,661; 6,545,197; and 6,107,539,

As mentioned above, the diaper pant 100P may also include an absorbent assembly 140 that is joined to the chassis 102. As shown in Figure 2A, the absorbent assembly 140 may have a laterally extending front edge 148 in the front waist region 116 and may have a longitudinally opposing and laterally extending back edge 150 in the back waist region 118. The absorbent assembly may have a longitudinally extending right side edge 152 and may have a laterally opposing and longitudinally extending left side edge 154, both absorbent assembly side edges 152 and 154 may extend longitudinally between the front edge 148 and the back edge 150. The absorbent assembly 140 may additionally include one or more absorbent cores 142 or absorbent core layers. The absorbent core 142 may be at least partially disposed between the topsheet 138 and the backsheet 136 and may be formed in various sizes and shapes that are compatible with the diaper. Exemplary absorbent structures for use as the absorbent core of the present disclosure are described in U.S. Patent Nos. 4,610,678; 4,673,402; 4,888,231; and 4,834,735,

Some absorbent core embodiments may comprise fluid storage cores that contain reduced amounts of cellulosic airfelt material. For instance, such cores may comprise less than about 40%, 30%, 20%, 10%, 5%, or even 1% of cellulosic airfelt material. Such a core may comprise primarily absorbent gelling material in amounts of at least about 60%, 70%, 80%, 85%, 90%, 95%, or even about 100%, where the remainder of the core comprises a microfiber glue (if applicable). Such cores, microfiber glues, and absorbent gelling materials are described in U.S. Patent Nos. 5,599,335; 5,562,646; 5,669,894; and 6,790,798 as well as U.S. Patent Publication Nos. 2004/0158212 A1 and 2004/0097895 A1,

As previously mentioned, the diaper 100P may also include elasticized leg cuffs 156. It is to be appreciated that the leg cuffs 156 can be and are sometimes also referred to as leg bands, side flaps, barrier cuffs, elastic cuffs or gasketing cuffs. The elasticized leg cuffs 156 may be configured in various ways to help reduce the leakage of body exudates in the leg regions. Example leg cuffs 156 may include those described in U.S. Patent Nos. 3,860,003; 4,909,803; 4,695,278; 4,795,454; 4,704,115; 4,909,803; and U.S. Patent Publication No. 2009/0312730 A1,

As mentioned above, diaper pants may be manufactured with a ring-like elastic belt 104 and provided to consumers in a configuration wherein the front waist region 116 and the back waist region 118 are connected to each other as packaged, prior to being applied to the wearer. As such, diaper pants may have a continuous perimeter waist opening 110 and continuous perimeter leg openings 112 such as shown in Figure 1. The ring-like elastic belt may be formed by joining a first elastic belt to a second elastic belt with a permanent side seam or with an openable and reclosable fastening system disposed at or adjacent the laterally opposing sides of the belts.

As previously mentioned, the ring-like elastic belt 104 may be defined by a first elastic belt 106 connected with a second elastic belt 108. As shown in Figures 2A and 2B, the first elastic belt 106 extends between a first longitudinal side edge 111a and a second longitudinal side edge 111b and defines first and second opposing end regions 106a, 106b and a central region 106c. And the second elastic 108 belt extends between a first longitudinal side edge 113a and a second longitudinal side edge 113b and defines first and second opposing end regions 108a, 108b and a central region 108c. As measured in an extended state, the distance between the first longitudinal side edge 111a and the second longitudinal side edge 111b defines the pitch length, PL, of the first elastic belt 106, and the distance between the first longitudinal side edge 113a and the second longitudinal side edge 113b defines the pitch length, PL, of the second elastic belt 108. The central region 106c of the first elastic belt is connected with the first waist region 116 or first end region 116a of the chassis 102, and the central region 108c of the second elastic belt 108 is connected with the second waist region 118 or second end region 118a of the chassis 102. As shown in Figure 1, the first end region 106a of the first elastic belt 106 is connected with the first end region 108a of the second elastic belt 108 at first side seam 178, and the second end region 106b of the first elastic belt 106 is connected with the second end region 108b of the second elastic belt 108 at second side seam 180 to define the ring-like elastic belt 104 as well as the waist opening 110 and leg openings 112. It is to be appreciated that the first belt 106 and the second belt 108 may be permanently or refastenably connected with each other at the first side seam 178 and the second side seam 180. The side seams 178, 180 may comprise a permanent bond, such as a thermal, pressure, or adhesive bond, or may be a releasable bond, such as a mechanical or cohesive fastener.

As shown in Figures 2A and 2B, the first elastic belt 106 also defines an outer laterally extending edge 107a and an inner laterally extending edge 107b, and the second elastic belt 108 defines an outer laterally extending edge 109a and an inner laterally extending edge 109b. The outer edge 107a of the first belt 106 is positioned longitudinally outward of the inner edge 107b, and the outer edge 109a of the second belt 108 is positioned longitudinally outward of the inner edge 109b. As such, as shown in Figure 1, a perimeter edge 112a of one leg opening may be defined by portions of the inner laterally extending edge 107b of the first elastic belt 106, the inner laterally extending edge 109b of the second elastic belt 108, and the first longitudinal or right side edge 128 of the chassis 102. And a perimeter edge 112b of the other leg opening may be defined by portions of the inner laterally extending edge 107b, the inner laterally extending edge 109b, and the second longitudinal or left side edge 130 of the chassis 102. The outer laterally extending edges 107a, 109a may also define the front waist edge 121 and the laterally extending back waist edge 122 of the diaper pant 100P.

It is to be appreciated that the first elastic belt 106 and the second elastic belt 108 may define different sizes and shapes. In some configurations, the first elastic belt 106 and/or second elastic belt 108 may define curved contours. For example, the inner lateral edges 107b, 109b of the first and/or second elastic belts 106, 108 may include non-linear or curved portions in the first and second opposing end regions. Such curved contours may help define desired shapes to leg opening 112, such as for example, relatively rounded leg openings. In addition to having curved contours, the elastic belts 106, 108 may include elastic strands 168 that extend along non-linear or curved paths that may correspond with the curved contours of the inner lateral edges 107b, 109b.

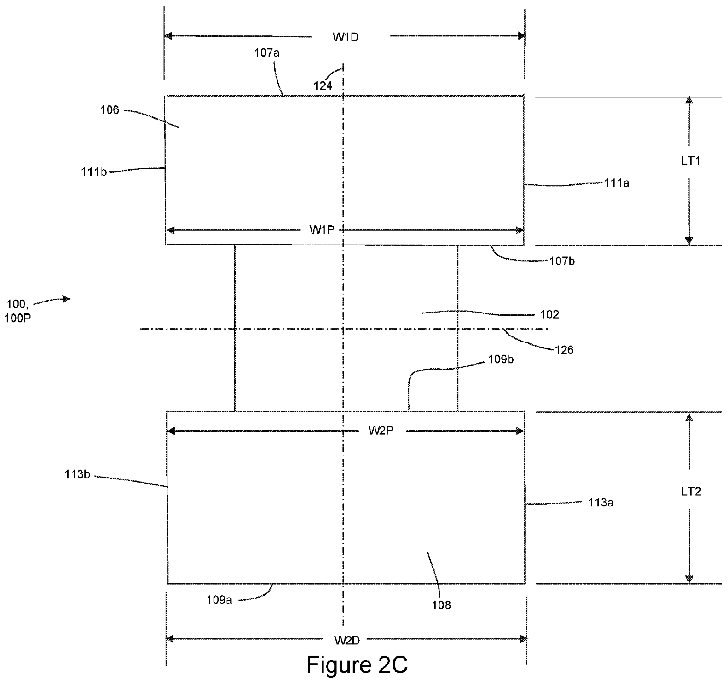

Figure 2C shows a configuration wherein the first elastic belt 106 and the second elastic belt 108 both define generally rectangular shapes. For example, as shown in Figure 2C, the outer laterally extending edge 107a of the first elastic belt 106 may comprise a lateral width of W1D and the inner laterally extending edge 107b may comprise a lateral width of W1P, wherein W1D and W1P are equal or substantially equal. In addition, the outer laterally extending edge 109a of the second elastic belt 108 may comprise a lateral width of W2D and the inner laterally extending edge 109b may comprise a lateral width of W2P, wherein W2D and W2P are equal or substantially equal.

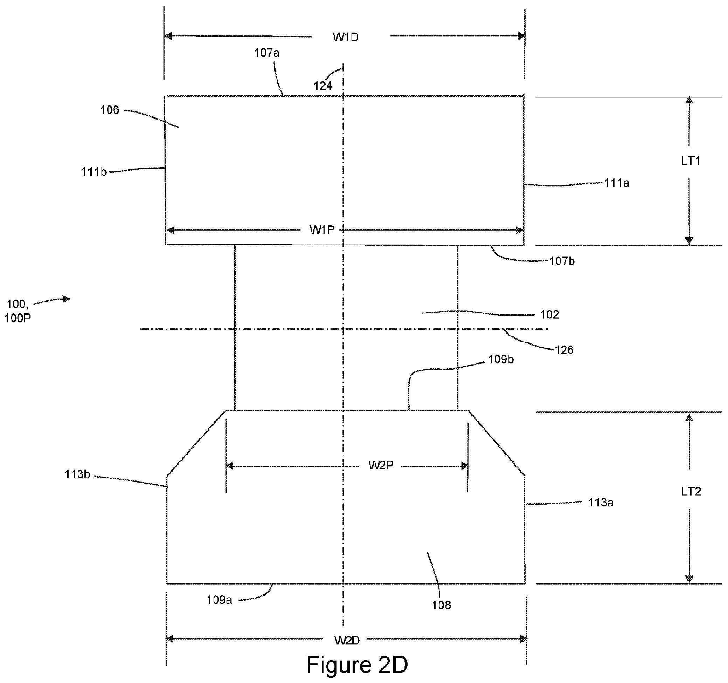

In some configurations, at least one of the first elastic belt 106 and the second elastic belt 108 may comprise lateral edges having different lengths. For example, Figure 2D shows a configuration wherein the first elastic belt 106 defines a generally rectangular shape, such as described with reference to Figure 2C, and wherein the outer laterally extending edge 109a of the second elastic belt 108 and the inner laterally extending edge 109b have different lengths. As shown in Figure 2D, the outer laterally extending edge 109a of the second elastic belt 108 may comprise a lateral width of W2D and the inner laterally extending edge 109b may comprise a lateral width of W2P, wherein W2D is greater than W2P.

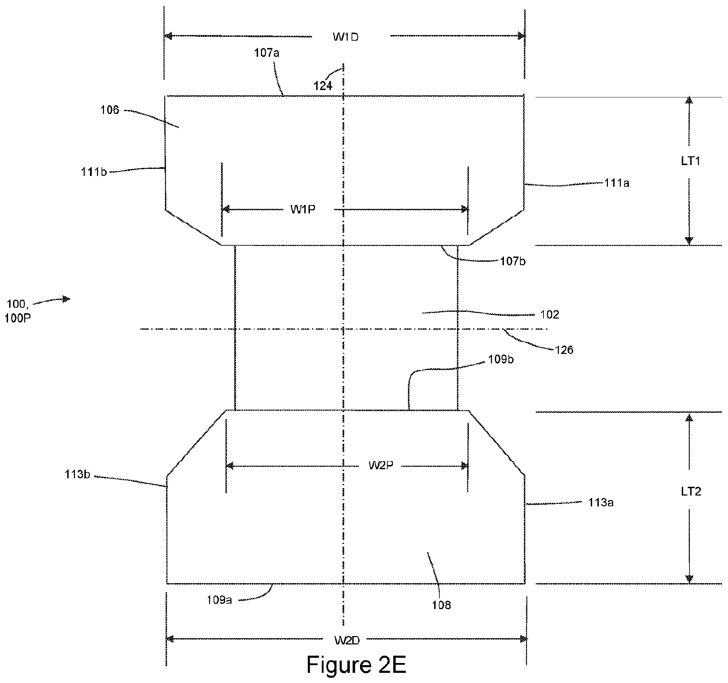

In some configurations, both the first elastic belt 106 and the second elastic belt 108 may comprise lateral edges having different lengths. For example, Figure 2E shows a configuration wherein the outer laterally extending edge 107a of the first elastic belt 106 and the inner laterally extending edge 107b have different lengths, and wherein the outer laterally extending edge 109a of the second elastic belt 108 and the inner laterally extending edge 109b have different lengths. As shown in Figure 2E, the outer laterally extending edge 107a of the first elastic belt 107 may comprise a lateral width of W1D and the inner laterally extending edge 107b may comprise a lateral width of W1P, wherein W1D is greater than W1P, and wherein the outer laterally extending edge 109a of the second elastic belt 108 may comprise a lateral width of W2D and the inner laterally extending edge 109b may comprise a lateral width of W2P, wherein W2D is greater than W2P.

With reference to Figures 2C-2E, the first elastic belt 106 may define a longitudinal length LT1 extending between outer laterally extending edge 107a and the inner laterally extending edge 107b, and the second elastic belt 108 may define a longitudinal length LT2 extending between outer laterally extending edge 109a and the inner laterally extending edge 109b. In some configurations, LT1 may be equal to LT2. In some configurations, LT1 may be less or greater than LT2. With continued reference to Figures 2C-2E, in some configurations, W1D may be equal to W1P, or W1D may be different than W1P. In some configurations, W2D may be equal to W2P, or W2D may be different than W2P. In some configurations, W1D and/or W1P may be equal to or different W2D and/or W2P.

With reference to Figures 2A, 2B, and 3, the first elastic belt 106 and the second elastic belt 108 may also each include a first substrate 162 and a second substrate 164. The first substrates 162 may be oriented to define at least a portion of a garment facing surface 115a of the first elastic belt 106 and a garment facing surface 117a the second elastic belt 108, and the second substrates 164 may be oriented to define at least a portion of a wearer facing surface 115b of the first elastic belt 106 and a wearer facing surface 117b of the second elastic belt 108. The first substrate 162 may extend from a proximal edge 162b to a distal edge 162a for a maximum length L1, and the second substrate 164 may extend from a proximal edge 164b to a distal edge 164a for a maximum length L2. It is to be appreciated that the distal edge 162a and/or the proximal edge 162b of the first substrate 162 may be straight and/or curved and/or may be parallel or unparallel to each other. It is also to be appreciated that the distal edge 164a and/or the proximal edge 164b of the second substrate 164 may be straight and/or curved and/or may be parallel or unparallel to each other. As such, the maximum length L1 refers to the longest distance extending longitudinally between the distal edge 162a and the proximal edge 162b of the first substrate 162, and the maximum length L2 refers to the longest distance extending longitudinally between the distal edge 164a and the proximal edge 164b of the second substrate 164. In some configurations, L1 may be equal to, less than, or greater than L2. In some configurations, L1 may be equal to or less than LT1, and L2 may be equal to or less than LT2. In some configurations, the distal edge 162a of the first substrate 162 may define at least a portion of the front waist edge 121 and/or at least a portion of back waist edge 122, and/or the distal edge 164a of the second substrate 164 may define at least a portion of the front waist edge 121 and/or at least a portion of back waist edge 122. As such, in some configurations, the distal edge 162a of the first substrate 162 and/or the distal edge 164a of the second substrate 164 may define at least a portion of the waist opening 110.

It is also to be appreciated that the first substrate 162 and/or the second substrate 164 may extend continuously from the first belt 106 to the second belt 108. For example, the first substrate 162 may be configured to define a continuous outer cover 162' that extends contiguously from the first waist edge 121 to the second waist edge 122, such as shown in Figures 1A, 2F, and 3C. It is also to be appreciated that diaper pants 100P with continuous outer covers, such as shown in Figures 1A, 2F, and 3C may also be configured to include various aspects of the frangible pathways and fastener components discussed herein.

It is to be appreciated that the first substrate 162 and the second substrate 164 may define various lateral widths that may or may not be equal. For example, as shown in Figure 2B, the first substrate 162 may extend laterally between a first longitudinal edge 162e and a second longitudinal edge 162f to define a first lateral width W1, and the second substrate 164 may extend laterally between a first longitudinal edge 164e and a second longitudinal edge 164f to define a second lateral width W2.

In some configurations, the proximal edge 162b of the first substrate 162 and/or the proximal edge 164b of the second substrate 164 may extend laterally across the backsheet 136. As shown in Figures 2A-3, the first substrate 162 includes a garment facing surface 162c and an opposing wearer facing surface 162d, and the second substrate 164 includes a garment facing surface 164c and an opposing wearer facing surface 164d.

In some configurations, the first elastic belt 106 and/or the second elastic belt 108 may include a folded portion of at least the first substrate 162 and/or the second substrate 164. For example, as shown in Figures 3A and 3B, the first elastic belt 106 and/or the second elastic belt 108 may include a folded portion 162g of the first substrate 162 extending longitudinally between a fold line 162h in the first substrate 162 and a lateral edge 162i. As such, the folded portion 162g of the first substrate 162 may be connected with the wearer facing surface 164d of the second substrate 164. In some configurations, the folded portion 162g of the first substrate 162 may also be connected with and/or overlap the chassis 102. In some configurations, the folded portion 162g of the first substrate 162 may also be connected with the wearer facing surface 162d of the first substrate 162. In some configurations, a portion of the folded portion 162g of the first substrate 162 may be left unbonded to the chassis 102 and/or the second substrate 164, forming a pocket having an opening oriented toward the lateral centerline 162c of the chassis 102. In another example, the first elastic belt 106 and/or the second elastic belt 108 may include a folded portion of the second substrate 164 extending longitudinally between a fold line in the second substrate 164 and a lateral edge. As such, the folded portion of the second substrate 164 may be connected with the garment facing surface 162c of the first substrate 162. As such, in some configurations, a fold line of the first substrate 162 and/or a fold line of the second substrate 164 may define at least a portion of the waist opening 110. It is to be appreciated that various waist configurations may be utilized. For example, as shown in Figure 3A1, the folded portion 162g may be sandwiched between the second substrate 164 and the backsheet 136. In another example shown in Figure 3A2, the second substrate 164 may be sandwiched between the folded portion 162g and the backsheet 136. Although Figures 3A1 and 3A2 show configurations of the first belt 106, it is to be appreciated that such configurations may be applied with the second belt 108.

It is to be appreciated that the first elastic belt 106 and the second elastic belt 108 may comprise the same materials and/or may have the same structure. In some embodiments, the first elastic belt 106 and the second elastic belt may comprise different materials and/or may have different structures. It should also be appreciated that components of the first elastic belt 106 and the second elastic belt 108, such as the first substrate 162, and/or second substrate 164 may be constructed from various materials. For example, the first and/or second belts may include a first substrate 162, and/or second substrate 164 that may be manufactured from materials such as plastic films; apertured plastic films; woven or nonwoven webs of natural materials (e.g., wood or cotton fibers), synthetic fibers (e.g., polyolefins, polyamides, polyester, polyethylene, or polypropylene fibers) or a combination of natural and/or synthetic fibers; or coated woven or nonwoven webs. In some configurations, the first and/or second belts may include a first substrate 162, and/or second substrate 164 comprising a nonwoven web of synthetic fibers, and may include a stretchable nonwoven. In some configurations, the first and second elastic belts may include an inner hydrophobic, non-stretchable nonwoven material and an outer hydrophobic, non-stretchable nonwoven material. It is to be appreciated that the belts may configured in various ways, such as disclosed for example, in U.S. Patent Publication No. 2022/0142828 A1 and Chinese Patent Application No. CN2021/077843,

One or more substrates of the belt may include fibers. The characteristics of these fibers may be the same or different in the outer garment facing surface and the inner wearer facing surface of the belt. Further, the characteristics of these fibers may be present in one or more the substrate layers forming the belt. Those fibers may be spunbond, carded, wet-laid, meltblown, hydroentangled, hydrojetted, or otherwise processed as is known in the art. The nonwoven may be a composite material such as a spunbond-meltblown-spunbond (SMS) material. For example, at least one of the outer garment facing surface and the inner wearer facing surface of the substrate may comprise carded nonwoven material. At least one of the outer garment facing surface and the inner wearer facing surface of the belt may comprise a substrate having a plurality of fibers that are hydroentangled or have undergone a hydrojetting process. Additionally, the substrate may include a plurality of fibers having a denier or less than about 1.5 or less than about 1.25 or less than about 1, specifically reciting all 0.1 increments within the above-recited range and all ranges formed therein or thereby.

The fibers of the substrates of the present disclosure may comprise mono-component fibers or multi-component fibers, such as bi-component fibers or tri-component fibers, for example. Multi-component fibers, as used herein, means fibers comprising more than one chemical species or material (i.e., multi-constituent fibers). The fibers may comprise petroleum sourced resins, recycled resins, and/or bio-sourced resins, such as polylactic acid from Nature Works, polyethylene and/or polypropylene from Braskem, and polybutylene terephthalate from Lanxess. The fibers may have round, triangular, tri-lobal, or otherwise shaped cross-sections, for example. Often, the different polymer components have different melting temperatures, viscosities, glass transition temperatures, crystallinities, and/or crystallization rates. The multi-component fibers, such as bi-component fibers, may comprise sheath/core, side-by-side, islands in the sea, and/or eccentric configurations or may have other configurations. As an example, in the context of bi-component fibers, fibers comprising a core/sheath configuration may be comprised of a first polymer forming the core of the fiber, and a second polymer partially or completely surrounding the first polymer and forming the sheath of the fiber. For example, the substrate may comprise a polypropylene-polyethylene side by side bicomponent fibers.

The fibers of the substrates of the present disclosure may comprise crimped fibers. These crimped fibers may be included in at least one of the outer garment facing surface and the inner wearer facing surface of the belt. Crimped fibers may result when the different polymer components of multi- component fibers have different melting temperatures, viscosities, glass transition temperatures, crystallinities, and/or crystallization rates, and are disposed in an eccentric sheath/core or side-by- side configuration within the fibers. As the multi-component fibers cool after formation, a first polymer component may solidify and/or shrink at a faster rate than a second polymer component while the second polymer component may have sufficient rigidity to resist compression along a longitudinal fiber axis. The continuous fibers may deform and curl up when strain on the fiber is relieved, thereby causing what is known as "crimp" in the fibers. Crimp of the fibers aids in the softness and loft of a nonwoven web, which is consumer desirable. Additional details for fibers suitable for inclusion in the substrates discussed herein is disclosed in U.S. Patent Publication No. 2020/0337910, which is incorporated by reference herein.

Elastic material 167 may be positioned between the wearer facing surface 162d of the first substrate 162 and the garment facing surface 164c of the second substrate 164. It is to be appreciated that the elastic material 167 may include one or more elastic elements such as strands, ribbons, elastic films, or panels extending along the lengths of the elastic belts. As shown in Figures 2A and 3, the elastic material 167 may include a plurality of elastic strands 168. In some configurations, the elastic material 167 may be an elastic film used to form a zero-strain elastic laminate comprising an elastic film bonded to one or more nonwoven layers and subsequently subjected to mechanical deformation or activation sufficient to weaken the nonwoven layer(s) and enable the laminate to stretch and recover elastically.

It is also to be appreciated that the first substrate 162, second substrate 164, and/or elastic material 167 of the first elastic belt 106 and/or second elastic belt 108 may be bonded together and/or with other components, such as the chassis 102, with adhesive and/or mechanical bonds. It is to be appreciated that adhesive and mechanical bonding methods may be utilized alone or in combination with each other.

In some configurations, adhesive may be applied to at least one of the first substrate 162, second substrate 164, and/or elastic material 167 when being combined to form the first elastic belt 106 and/or second elastic belt 108. In some configurations, mechanical bonding devices may apply mechanical bonds to the to at least one of the first substrate 162, second substrate 164, and/or elastic material 167 when being combined to form the first elastic belt 106 and/or second elastic belt 108. Such mechanical bonds may be applied with heat, pressure, and/or ultrasonic devices. In some configurations, mechanical bonding devices may apply bonds that bond the first substrate 162, second substrate 164, and/or elastic material 167 together and/or may act to trap or immobilize discrete lengths of the contracted elastic strands in the first elastic belt 106 and/or second elastic belt 108.

It is also to be appreciated that the first substrate 162, second substrate 164, and/or elastic material 167 may be bonded together with various methods and apparatuses to create various elastomeric laminates, such as described in U.S. Patent Publication Nos. 2018/0168878 A1; 2018/0168877 A1; 2018/0168880 A1; 2018/0170027 A1; 2018/0169964 A1; 2018/0168879 A1; 2018/0170026 A1; 2018/0168889 A1; 2018/0168874 A1; 2018/0168875 A1; 2018/0168890 A1; 2018/0168887 A1; 2018/0168892 A1; 2018/0168876 A1; 2018/0168891 A1; 2019/0070042 A1; 2019/0070041 A1; 2021/0282797 A1; and 2021/0275362 A1, and combinations thereof,

It is to be appreciated that components of the first elastic belt 106 and/or the second elastic belt 108 may be assembled in various ways and various combinations to create various desirable features that may differ along the lateral width and/or longitudinal length of the first elastic belt 106 and/or the second elastic belt 108. Such features may include, for example, Dtex values, bond patterns, aperture arrangements, elastic positioning, Average Dtex values, Average Pre-Strain values, rugosity frequencies, rugosity wavelengths, height values, and/or contact area. It is to be appreciated that differing features may be imparted to various components, such as for example, the first substrate 162, second substrate 164, and elastic material 167 before and/or during stages of assembly of the first elastic belt 106 and/or the second elastic belt 108.

It is to be appreciated that the first elastic belt 106 and/or the second elastic belt 108 may include various configurations of belt elastic materials 167 arranged in relation to each other and to the first substrate 162, and the second substrate 164. As discussed above, the elastic material 167 may include configurations of one or more elastic elements such as strands, ribbons, films, or panels positioned in various arrangements. In some configurations, the elastic material 167 may comprise various elastics, elastic features and arrangements, and processes for assembly, such as described in 2018/0168889 A1; 2018/0168874 A1; 2018/0168875 A1; 2018/0168890 A1; 2018/0168887 A1; 2018/0168892 A1; 2018/0168876 A1; 2018/0168891 A1; 2019/0298586 A1; 2019/0070042 A1; 2018/0168878 A1; 2018/0168877 A1; 2018/0168880 A1; 2018/0170027 A1; 2018/0169964 A1; 2018/0168879 A1; 2018/0170026 A1; 2019/0070041 A1; 2021/0282797A1; and 2021/0275362 A1, It is also to be appreciated the elastic materials 167 herein may be configured with identical or different colors in various different locations on the first elastic belt 106 and/or the second elastic belt 108.

In some configurations, the elastic material 167 may be configured as elastic strands 168 disposed at a constant interval in the longitudinal direction. In other embodiments, the elastic strands 168 may be disposed at different intervals in the longitudinal direction. In some configurations, the Dtex values of the elastic strands 168 may be constant or varied along the longitudinal direction. In some configurations, the elastic material 167 in a stretched condition may be interposed and joined between uncontracted substrate layers. When the elastic material 167 is relaxed, the elastic material 167 returns to an unstretched condition and contracts the substrate layers. The elastic material 167 may provide a desired variation of contraction force in the area of the ring-like elastic belt. It is to be appreciated that the chassis 102 and elastic belts 106, 108 may be configured in different ways other than as depicted in attached Figures. It is also to be appreciated that the elastic material 167 may be joined to the substrates continuously or intermittently along the interface between the elastic material 167 and the substrates. In some configurations, the elastic strands 168 may be in the form of extruded elastic strands, which may also be bonded with the first substrate 162 and/or second substrate 164 in a pre-corrugated configuration, such as disclosed for example in U.S. Patent No. 5,681,302,

As discussed above for example with reference to Figures 2A and 3, the elastic material 167 discussed herein may be in the form of elastic strands 168. In some configurations, the elastic strands 168 may be parallel with each other and/or with the lateral axis 126. It is to be appreciated that the first elastic belt 106 and/or second elastic belt 108 may be configured to include various quantities of elastic strands 168. In some configurations, elastic strands 168 may be grouped in pairs. In some configurations, the first elastic belt 106 and/or second elastic belt 108 may comprise from about 10 to about 1500 elastic strands 168. It is also to be appreciated that elastic strands 168 herein may comprise various Dtex values, strand spacing values, and pre-strain values and such elastic strands 168 may utilized with other elastic strands to create first and second elastic belts 106, 108 comprising elastic strands 168 in various combinations of Dtex values, strand spacing values, and pre-strain values. For example, in some configurations, the Average-Dtex of one or more elastic strands 168 may be greater than 500. In some configurations, the Average-Dtex of one or more elastic strands 168 may be from about 10 to about 1500, specifically reciting all 1 Dtex increments within the above-recited range and all ranges formed therein or thereby. In some configurations, a plurality of elastic strands 168 may comprise an Average-Strand-Spacing of less than or equal to 4 mm. In some configurations, a plurality of elastic strands 168 may comprise an Average-Strand-Spacing from about 0.25 mm to about 4 mm, specifically reciting all 0.01 mm increments within the above-recited range and all ranges formed therein or thereby. In some configurations, a plurality of elastic strands 168 may comprise an Average-Strand-Spacing of greater than 4 mm. In some configurations, the Average-Pre-Strain of each of a plurality of elastic strands may be from about 50% to about 400%, specifically reciting all 1% increments within the above-recited range and all ranges formed therein or thereby. In some configurations, the elastic strands 168 comprise an Average-Strand-Spacing from about 0.25 mm to about 4 mm and an Average-Dtex from about 10 to about 500. In some configurations, the elastic strands 168 may comprise an Average-Pre-Strain from about 75% to about 300%.

In some configurations, a first plurality of elastic strands may comprise a first Average-Pre-Strain from about 75% to about 300%, and a second plurality of elastic strands may comprise a second Average-Pre-Strain that is greater than first Average-Pre-Strain. In some configurations, a first plurality of elastic strands comprises an Average-Strand-Spacing from about 0.25 mm to about 4 mm and an Average-Dtex from about 10 to about 500; and a second plurality of elastic strands may comprise an Average-Strand-Spacing greater than about 4 mm and an Average-Dtex greater than about 450.

In some configurations, such as shown in Figure 2A, the elastic strands 168 may be referred to herein as outer waist elastics 170 and inner waist elastics 172. Elastic strands 168, such as the outer waist elastics 170, may continuously extend laterally between the first and second opposing end regions 106a, 106b of the first elastic belt 106 and between the first and second opposing end regions 108a, 108b of the second elastic belt 108. Some elastic strands 168, such as the inner waist elastics 172, may be configured with discontinuities in areas, such as for example, where the first and second elastic belts 106, 108 overlap portions of the chassis 102, such as the absorbent assembly 140.

As shown in Figure 2A, the first elastic belt 106 and/or the second elastic belt 108 may be configured with low-stretch zones 701 and high-stretch zones 703. The first elastic belt 106 and/or the second elastic belt 108 may include a first high-stretch zone 703a and a second high-stretch zone 703b separated laterally by a low-stretch zone 701. Portions of the chassis 102, such as the backsheet 136 and absorbent assembly 140, may be connected with the first elastic belt 106 and/or the second elastic belt 108 in the low-stretch zones 701 in the first waist region 116 and/or the second waist region 118. The high-stretch zones 703 are elasticated by the elastic material 167, such as the elastic strands 168, 172; and the low-stretch zones 701 may comprise cut lines separating the elastic material 167, such as the elastic strands 168, 172. In some configurations, the elastic material 167 may be cut in an unbonded region where the elastic material is not bonded with first substrate 162 and the second substrate 164. Thus, the elastic material 167 retracts from the unbonded region and form low-stretch zone 701. In some configurations, the elastic material 167 may be cut into several discrete pieces. In turn, the low-stretch zones 701 define regions of the first elastic belt 106 and/or the second elastic belt 108 that have relatively less elasticity than the high-stretch zones 703. The discrete elastic material 167 that has been cut and which are elastically contracted do not add any substantial amount of elastication to the low-stretch zone 701. As such, upon application of a force, the high-stretch zones 703 will elongate more than the low-stretch zones 701. As provided above, the terms "elastic," "elastomer" or "elastomeric" refers to materials exhibiting elastic properties, which include any material that upon application of a force to its relaxed, initial length can stretch or elongate to an elongated length more than 10% greater than its initial length and will substantially recover back to about its initial length upon release of the applied force. In some configurations, the first elastic belt 106 and/or the second elastic belt 108 may be configured with high-stretch zones 703 that are elastic and may be configured with low-stretch zones 701 that are not elastic or "inelastic."

As discussed above, the diaper pants 100P described with reference to Figures 1-3C may include one or more frangible pathways in the first belt 106 and/or the second belt 108. For example, Figures 4, 5A and 5B show an example diaper pant 100P with a first belt 106 that includes frangible pathways 700. The frangible pathways 700 may be configured to allow the first elastic belt 106 to be relatively easily torn along the frangible pathway 700, such as when removing the diaper pant 100P from a wearer. Figure 5A shows a view of the diaper pant 100P from Figure 4, illustrating the first belt 106 after having been torn along the frangible pathway 700 through both the outer longitudinal outer laterally extending edge 107a and the inner laterally extending edge 107b of the first belt 106. As such, the first elastic belt 106 shown in Figure 5A is separated by opposing tear lines 705. It is to be appreciated the first elastic belt 106 may be torn along both frangible pathways 700 in Figures 5A. For example, Figure 5B shows the diaper pant of Figure 4 showing the front belt having been torn along two frangible pathways 700. As shown in Figure 5B, the central region 106c of the first elastic belt 106 may remain bonded with the chassis 102 after separating the first and second opposing end regions 106a, 106b from the central region 106c by tearing the elastic belt 106 along the frangible pathways 700.

As discussed in more detail below, the frangible pathways 700 may comprise a plurality of lines of weakness 704 configured such that all elastic strands 168 in the first elastic belt 106 are severed at least once in the frangible pathway 700. Severing the elastic strands 168 in the frangible pathway 700 helps make it relatively easier to tear the first elastic belt 106 along the frangible pathway 700. For example, when the elastic strands 168 are severed, the first substrate 162 and second substrate 164 of the first elastic belt 106 need only need to be torn without having to also tear uncut elastic strands 168. It is to be appreciated that the diaper pant 100P may include various quantities of frangible pathways 700 that may be: positioned in various locations; define various shapes; and extend for various lengths. For example, the first elastic belt 106 may comprise a first belt length defined by a longitudinal distance between the proximal edge 107b and the distal edge 107a, and the frangible pathway 700 may extend for a total length from an outermost edge of a line of weakness 704 nearest the proximal edge 107b of the first belt 106 to an outermost edge of a line of weakness 704 nearest the distal edge 107a of the first belt 106. In some configurations, the frangible pathway 700 may extend for a total length that is greater than, equal to, or less than the first belt length. In some configurations, the lines of weakness 704 may extend for a length from a first end to a second end, and a sum of the all the lengths of lines of weakness 704 in the frangible pathway 700 may be greater than the frangible pathway total length.

In some configurations, diaper pants 100P may be configured such that one or both of the first elastic belt 106 and the second elastic belt 108 include one or more frangible pathways 700. The frangible pathways 700 may be positioned in various locations on the first and second elastic belts 106, 108. For example, such as shown in Figures 4, 5A and 5B, frangible pathways 700 may extend to overlap with the chassis 102. In some configurations, the frangible pathways 700 may extend in straight lines and/or may be curved and/or have curved portions. In some configurations, the frangible pathways 700 may extend longitudinally for the entire length or less than the entire length of the first belt 106 and/or second belt 108. In some configurations, frangible pathways 700 may be positioned partially or entirely laterally between the first and second side seams 178, 180 and the chassis 102.

In some configurations, the frangible pathways 700 may be configured and/or positioned to provide access to and/or function with other features, such as disposal features. For example, the diaper pant 100P shown in Figures 4, 5A and 5B includes fastener components 707 positioned on the wearing facing surface 115b of the first elastic belt 106. In some configurations, the fastener components 707 may be positioned between the first elastic belt 106 and the chassis 102. The fastener component 707 may be configured to refastenably connect with other portions of the diaper pant 100P, such as for example, the garment facing surfaces of the first elastic belt 106, the second elastic belt 108, or the chassis 102. As such, once the first elastic belt 106 is torn along the frangible pathways 700, the diaper pant 100P may be removed from a wearer and rolled or folded up for disposal, and the fastener component 707 may be connected with another portion of the diaper pant 100P to help maintain the diaper pant 100P in a disposal configuration.

It is also to be appreciated that the fastener component 707 may be configured in various ways, such as hooks, loops, and/or adhesive. For example, the fastener component 707 may comprise hook elements or adhesive adapted to refastenably connect with another surface of the diaper pant 100P. In some configurations, the fastener component 707 may comprise loop elements adapted to refastenably connect with hook surface on the diaper pant 100P. The fastener component 707 may be a separate element connected with the elastic belt 106 in various ways, such as mechanical bonding, adhesive bonding, or both. In some configurations, the fastener component 707 may be integrally formed from materials of the elastic belt 106, 108. In some configurations, the fastener component 707 may be printed and/or comprise materials of various different colors such that the fastener component 707 may be visible from outside the diaper pant 100P.

As previously mentioned, the fastener component 707 may comprise a hook material that can refastenably engage with substrates, such as nonwovens for example, on an exterior surface of the diaper pant 100P. For example, the fastener component 707 may comprise a substrate comprising hooks, with the substrate bonded to the elastic belt 106, 108, such as the second substrate 164, which may be in the form of a nonwoven. It is to be appreciated that the substrate may be bonded to the elastic belt 106, 108 in various ways, such as for example, with mechanical bonds, thermal bonds, ultrasonic bonds, and/or adhesive bonds or combinations thereof. In some configurations, hooks may be integrally formed from the second substrate 164, which may be in the form of a nonwoven. The fastener component 707 may comprise one material or a combination of two or more materials arranged in at least partially overlapping configuration. In some configurations, the fastener component 707 may comprise other fastener types as known in the art.

It is to be appreciated that the fastener component 707 may comprise any of a wide variety of shapes, including rectangles or other polygons, circles, ovals, shapes having exterior convexities or concavities or combinations thereof, or one or a plurality of lines or geometric shapes forming an array. It is to be appreciated that the fastener component 707 may comprise various sizes. For example, in some configurations, the fastener component 707 may have a lateral width of between about 5 mm and about 100 mm, specifically reciting all 0.1 mm increments within the above-recited range and all ranges formed therein or thereby. In some configurations, the fastener component 707 may have a longitudinal length of between about 10 mm and about 100 mm, specifically reciting all 0.1 mm increments within the above-recited range and all ranges formed therein or thereby. The fastener component 707 may be aligned parallel the lateral centerline 126a, 126b of the elastic belt 106, 108 or may be oriented at an angle relative the longitudinal centerline 126a, 126 of the elastic belt 106, 108 of between 0 and 90 degrees. The fastener component 707 may comprise an array of two or more spaced-apart fastening elements. The fastener component 707 may have a color that is visible through any layers of the elastic belt 106, 108 on which the fastener component 707 is located. The elastic belt 106, 108 and/or chassis 102 may include printing or other indicia highlighting to a caregiver the location, function, and/or usage of the fastener component 707. The bond, or bond pattern, attaching the fastener component 707 to the elastic belt 106, 108 may be visually or tactilely distinct from the surrounding belt material in order to provide the caregiver a signal or a mechanical grip advantage.

It is also to be appreciated that the frangible pathways 700 may comprise lines of weakness 704 that are: configured in various ways; positioned in various locations and orientations relative to each other; defined by various shapes; and extending for various lengths. For example, in some configurations, the lines of weakness 704 may comprise discrete cut lines that penetrate through some or all the layers of the elastic belt 106. In some configurations, the lines of weakness 704 comprise discrete bonds wherein materials of the first substrate and the second substrate are fused together. In some configurations, the lines of weakness 704 may be linear, curvilinear, or have a regular or irregular geometry and may comprise one or more of a perforation, a bond, an aperture, or a mechanically thinned region of a material such as a nonwoven, or a combination thereof. It is also to be appreciated that the lines of weakness 704 can be formed with different lengths and spacings to achieve different separation forces.

As discussed above, absorbent articles 100, such as diaper pants 100P, may be configured with frangible pathways 700 comprising lines of weakness 704 arranged in various ways to help improve a caregiver's ability to remove a soiled diaper pant 100P from a wearer without having to remove a soiled diaper pant from a wearer by sliding the soiled diaper pant down the wearer's legs. As discussed above, the frangible pathways 700 may be configured to allow the first elastic belt 106 and/or the second elastic belt 108 to be relatively easily torn along the frangible pathway 700, such as when removing the diaper pant 100P from a wearer. In addition, the frangible pathways 700 may also be configured to provide access to fastener components 707 that may be used to help hold a soiled product in a disposal configuration. The following provides a discussion of example implementations of frangible pathways 700 on diaper pants 100P in the context of the above description of various details of absorbent articles 100, fastener components 707, frangible pathways 700, and lines weakness 704. It is to be appreciated that discussions of frangible pathways 700 in the first elastic belt 106 herein may also apply to frangible pathways 700 in the second elastic belt 108.

It is to be appreciated that frangible pathways 700 may be positioned in various locations and/or orientations relative to other components of the absorbent article 100 and/or may be configured to function in various ways to help facilitate removal of diaper pant from a wearer. For example, the diaper pant 100P shown in Figures 6A and 6B may include one or more frangible pathways 700 extending between a distal terminus 808 on the outer edge 107a of the first belt 106 and a proximal terminus 810 on the inner edge 107b of the first belt 106. As illustrated in Figures 6A and 6B, the diaper pant 100P includes a first frangible pathway 700a and a second frangible pathway 700b in the first belt 106. The first frangible pathway 700a may extend between a first distal terminus 808a on the outer edge 107a of the first belt 106 and a first proximal terminus 810a on the inner edge 107b of the first belt 106. And the second frangible pathway 700b may extend between a second distal terminus 808b on the outer edge 107a of the first belt 106 and a second proximal terminus 810b on the inner edge 107b of the first belt 106. It is to be appreciated that the first and second frangible pathways 700a, 700b may comprise lines of weakness 704 as described above.

It is to be appreciated that the first distal terminus 808a and the second distal terminus 808b may be located in various lateral positions on the outer edge 107a of the first belt 106. For example, in some configurations, the first distal terminus 808a and/or the second distal terminus 808b may be positioned in the central region 106c of the first belt 106. In some configurations, the first distal terminus 808a and/or the second distal terminus 808b may be positioned laterally between the first longitudinal edge 128 and the second longitudinal edge 130 of the chassis 102. In some configurations, the first distal terminus 808a and/or the second distal terminus 808b may be positioned in the first end region 106a and/or the second end region 106b of the first belt 106. In some configurations, the first distal terminus 808a and/or the second distal terminus 808b may be positioned laterally outboard of the first longitudinal edge 128 and the second longitudinal edge 130 of the chassis 102. In some configurations, the first distal terminus 808a and/or the second distal terminus 808b may be positioned laterally between the first longitudinal edge 128 of the chassis 102 and the first side seam 178 and/or may be positioned laterally between the second longitudinal edge 130 of the chassis 102 and the second side seam 180. In some configurations, the first distal terminus 808a may be laterally aligned with the first longitudinal edge 128 of the chassis 102 or the first longitudinal side edge 111a of the first belt 106. In some configurations, the first distal terminus 808a may be positioned laterally between the first longitudinal edge 128 of the chassis 102 and the first longitudinal side edge 111a of the first belt 106. In some configurations, the second distal terminus 808b may be laterally aligned with the second longitudinal edge 130 of the chassis 102 or the second longitudinal side edge 111b of the first belt 106. In some configurations, the second distal terminus 808b may be positioned laterally between the second longitudinal edge 130 of the chassis 102 and the second longitudinal side edge 111b of the first belt 106.

It is also to be appreciated that the first proximal terminus 810a and the second proximal terminus 810b may be located in various lateral positions on the inner edge 107b of the first belt 106. For example, in some configurations, the first proximal terminus 810a and/or the second proximal terminus 810b may be positioned in the central region 106c of the first belt 106. In some configurations, the first proximal terminus 810a and/or the second distal terminus 810b may be positioned laterally between the first longitudinal edge 128 and the second longitudinal edge 130 of the chassis 102. In some configurations, the first proximal terminus 810a and/or the second proximal terminus 810b may be positioned in the first end region 106a and/or the second end region 106b of the first belt 106. In some configurations, the first proximal terminus 810a and/or the second proximal terminus 810b may be positioned laterally outboard of the first longitudinal edge 128 and the second longitudinal edge 130 of the chassis 102. In some configurations, the first proximal terminus 810a and/or the second proximal terminus 810b may be positioned laterally between the first longitudinal edge 128 of the chassis 102 and the first side seam 178 and/or may be positioned laterally between the second longitudinal edge 130 of the chassis 102 and the second side seam 180. In some configurations, the first proximal terminus 810a may be laterally aligned with the first longitudinal edge 128 of the chassis 102 or the first longitudinal side edge 111a of the first belt 106. In some configurations, the first proximal terminus 810a may be positioned laterally between the first longitudinal edge 128 of the chassis 102 and the first longitudinal side edge 111a of the first belt 106. In some configurations, the second proximal terminus 810b may be laterally aligned with the second longitudinal edge 130 of the chassis 102 or the second longitudinal side edge 111b of the first belt 106. In some configurations, the second proximal terminus 810b may be positioned laterally between the second longitudinal edge 130 of the chassis 102 and the second longitudinal side edge 111b of the first belt 106.

It is to be appreciated that the first distal terminus 808a and the second distal terminus 808b may be located in various longitudinal positions between the outer edge 107a and the inner edge 107b of the first belt 106. And the first proximal terminus 810a and the second proximal terminus 810b may be located in various longitudinal positions between the outer edge 107a and the inner edge 107b of the first belt 106. For example, in some configurations, such as shown in Figure 6B2 for example, the first distal terminus 808a and/or the first proximal terminus 810a may be located on the first side seam 178 at positions longitudinally inboard of the outer edge 107a and longitudinally outboard of the inner edge 107b of the first belt 106. Also, as shown in Figure 6B2, the second distal terminus 808b and/or the second proximal terminus 810b may be located on the second side seam 180 at positions longitudinally inboard of the outer edge 107a and longitudinally outboard of the inner edge 107b of the first belt 106. As such, completing the tearing process of the first belt 106 may also require tearing portions of the first and/or second side seams 178, 180.

With continued reference to Figure 6B, the first belt 106 may also comprise grip regions 801 providing a place where a user may grasp a portion of the first belt 106 and begin the process of tearing the first belt along the frangible pathway 700. The grip region 801 may comprise an accessibility opening 802 in the first belt 106 and may also comprise a fastener component 707 positioned adjacent the accessibility opening 802. The accessibility opening 802 may comprise slits and/or openings in the first belt 106 and may penetrate through some or all layers of the first belt 106. It is to be appreciated that such slits or openings may be curved and/or straight. The accessibility opening 802 may also be considered part of the frangible pathway 700.

As shown in Figure 6B, the diaper pant 100P may include a first grip region 801a including a first accessibility opening 802a and second grip region 801b including a second accessibility opening 802b in the first belt 106. The first and second accessibility openings 802a, 802b may be positioned between the outer edge 107a and the inner edge 107b of the first belt 106. The first and second accessibility openings 802a, 802b may also be positioned in the central region 106c of the first belt 106 and may be positioned between the first longitudinal edge 128, the second longitudinal edge 130 of the chassis 102, and the first lateral edge 144 of the chassis 102. In addition, a first fastener component 707a may be positioned adjacent the first accessibility opening 802a, and a second fastener component 707a may be positioned adjacent the second accessibility opening 802a. The first frangible pathway 700a may comprise a first tear zone 813a extending from the first accessibility opening 802a to the first distal terminus 808a and a second tear zone 813b extending from the first accessibility opening 802a to the first proximal terminus 810a. The second frangible pathway 700b may comprise a first tear zone 813a extending from the second accessibility opening 802b to the second distal terminus 808b and a second tear zone 813b extending from the second accessibility opening 802b to the second proximal terminus 810b.

It is to be appreciated that the frangible pathways 700 may comprise one or more functional zones. In turn, the frangible pathways may comprise transition zones that may operatively connect such zones to help facilitate propagation of a tear along the frangible pathway 700 from one zone to another zone. The lines of weakness in the transition zones may be of particular lengths and/or angles relative to lateral centerlines and row spacing to help provide desired propagation of material failure when, for example, removing a product from a wearer. It is to be appreciated that the lengths, angles, and spacings in transition zones may be different from those in adjacent lines of weakness.

As shown in Figure 6B for example, the first tear zone 813a of the first frangible pathway 700a may comprise a first initial tear zone 815a extending from the first accessibility opening 802a to a first transition zone 817a. In addition, the first tear zone 813a of the first frangible pathway 700a may comprise a first secondary tear zone 819a extending from the first transition zone 817a to the first distal terminus 808a. The first tear zone 813a of the first frangible pathway 700a may also comprise a second initial tear zone 815b extending from the first accessibility opening 802a to a second transition zone 817b. Further, the first tear zone 813a of the first frangible pathway 700a may comprise a second secondary tear zone 819b extending from the second transition zone 817b to the first proximal terminus 810a. The first transition zone 817a may operatively connect the first initial tear zone 815a with the first secondary tear zone 819a to help facilitate the propagation of the tear along the first frangible pathway 700a from first initial tear zone 815a to the first secondary tear zone 819a. With continued reference to Figure 6B, the first tear zone 813a of the second frangible pathway 700b may comprise a first initial tear zone 815a extending from the second accessibility opening 802b to a first transition zone 817a. In addition, the first tear zone 813a of the second frangible pathway 700b may comprise a secondary tear zone 819a extending from the first transition zone 817a to the second distal terminus 808b. The first tear zone 813a of the second frangible pathway 700b may also comprise a second initial tear zone 815b extending from the second accessibility opening 802b to a second transition zone 817b. Further, the first tear zone 813a of the second frangible pathway 700b may comprise a second secondary tear zone 819b extending from the second transition zone 817b to the second proximal terminus 810b. The second transition zone 817b may operatively connect the second initial tear zone 815b with the second secondary tear zone 819b to help facilitate the propagation of the tear along the second frangible pathway 700b from second initial tear zone 815b to the second secondary tear zone 819b.

The accessibility opening 802 may help provide a caregiver or wearer access to and/or to grasp the fastener component 707 in the grip region 801 with a finger or thumb. The caregiver or user may then pull on grip region 801 to begin tearing the first belt 106 on the frangible pathway 700. In some configurations, tear lines may simultaneously propagate along the first tear zone 813a and the second tear zone 813b laterally outward from the central region 106c of the first belt 106 toward the distal terminus 808 and the proximal terminus 810. As discussed in more detail below, the diaper pant 100P may also be configured such that a tear line propagating along the first tear zone 813a and a tear line propagating along the second tear zone 813b may reach the distal terminus 808 and the proximal terminus 810, respectively, simultaneously or approximately simultaneously. It is also to be appreciated that some diaper pants 100P herein may be configured to include a frangible pathway 700 that extends through or around the fastener component 707 without an accessibility opening. In turn, a user may pinch and/or pull the belt where the frangible pathway 700 is located at or adjacent the fastener component 707 to initiate the tearing process along the frangible pathway 700.

As shown in Figure 6B, the frangible pathways 700 may be configured to extend laterally inward from the from the distal terminus 808 and/or the proximal terminus 810. In turn, portions of the frangible pathway 700 may extend to define an angle that is less than 90 degrees with respect to the outer edge 107a and/or the inner edge 107b of the first belt 106. Portions of the frangible pathway may extend to define an angle that is less than about 90 degrees, or less than about 80 degrees, or less than about 75 degrees, or less than about 65 degrees, or less than about 45 degrees, or less than about 35 degrees, or less than about 25 degrees with respect to the outer edge 107a and/or the inner edge 107b of the first belt 106. Thus, the frangible pathway may define an overall length that is greater than a longitudinal length LT1 of the first belt 106 and/or the longitudinal length LT2 of the second belt 108 discussed above with reference to Figures 2C-2E.

As discussed above, the first elastic belt 106 and/or the second belt 108 may be relatively easily torn along the frangible pathway 700, such as when removing the diaper pant 100P from a wearer. In addition, the first belt 106 may be separable along the first frangible pathway 700a and the second frangible pathway 700b to define a first belt zone 831, a second belt zone 832, and a third belt zone 833 positioned laterally between the first and second belt zones 831, 832.

Referring now to Figures 6A and 6B, when removing a diaper pant 100P from a wearer, a user may grab the first belt 106 in the grip region 801 by inserting one or more fingers and/or a thumb through the accessibility opening 802 to grasp a portion of the first belt 106 and fastener component 707. For example, with reference to Figures 6B and 6C, a caregiver may insert a finger or thumb through the first accessibility opening 802a and grasp the first belt 106 and the first fastener component 707a with a first hand. The caregiver's opposing second hand may be used to help stabilize the wearer. For example, the caregiver's opposing second hand may apply a holding or stabilizing force to the wearer at the central region 106c of the first belt 106. The user's first hand may then exert a pulling force Fp on the first grip region 801a of the first belt 106 outward away from the wearer to initiate a tearing of the first belt 106 along the first frangible pathway 700a, such as shown in Figure 6C.

With continued reference to Figure 6C, a pulling force Fp (generally represented by an arrow) may be applied to the first grip region 801a in a direction generally toward the first end region 106a of the first belt 106 and/or outward away from the first belt 106 and the wearer. As the force Fp is applied, a first tear line 705a and a second tear line 705b may simultaneously propagate along the first tear zone 813a and the second tear zone 813b, respectively. The first tear line 705a may propagate from the first accessibility opening 802a along the first tear zone 813a of the first frangible pathway 700a in longitudinal and lateral directions partially through and adjacent to the first fastener component 707a and then in a direction D1 that is generally laterally and longitudinally outward from the central region 106c of the first belt 106 and toward the first distal terminus 808a in the first end region 106a of the first belt 106. Simultaneously, the second tear line 705b may propagate from the first accessibility opening 802a in longitudinal and lateral directions partially through and adjacent to the first fastener component 707a along the second tear zone 813b of the first frangible pathway 700a in a direction D2 that is generally laterally outward and longitudinally inward from the central region 106c of the first belt 106 and toward the first proximal terminus 810a in the first end region 106a of the first belt 106.

In some configurations, the first tear line 705a may propagate from the first accessibility opening 802a along the first initial tear zone 815a of the first frangible pathway 700a to the first transition zone 817a. From the first transition zone 817a, the first tear line 705a may then propagate along the first secondary tear zone 819a to the first distal terminus 808a. In addition, the second tear line 705b may propagate from the first accessibility opening 802a along the second initial tear zone 815b of the first frangible pathway 700a to the second transition zone 817b. From the second transition zone 817b, the second tear line 705b may then propagate along the second secondary tear zone 819b to the first proximal terminus 810a. As discussed in more detail below, the first frangible pathway 700a may be configured such that the first tear line 705a and the second tear line 705b may reach first distal terminus 808a and the first proximal terminus 810a, respectively, at the same time or about the same time.

As shown in Figure 6D, the first belt 106 may be separable along the first frangible pathway 700a to define a first belt zone 831. For example, the first belt zone 831 may be formed once the first tear line 705a propagates through the first distal terminus 808a and the second tear line 705b propagates through to the first proximal terminus 810a, the first belt zone 831 may be formed. As shown in Figure 6D, a first edge 831a of the first belt zone 831 is formed by tearing the first frangible pathway 700a. In addition, a first edge 833a of the third belt zone 833 discussed in more detail below is also formed by tearing the first frangible pathway 700a. The first belt zone 831 may extend from the first edge 831a of the first and second tear lines 705a, 705b to the first side seam 178 or the first longitudinal side edge 111a of the first belt 106. In addition, the first belt zone 831 may include the first fastener component 707a. As discussed below, the first belt zone 831 may include the entirety of or a portion of first fastener component 707a.

With the first belt zone 831 being defined by tearing the first belt 106 along the first frangible pathway 700a, a user may proceed to define the second belt zone 832 by tearing the first belt 106 along the second frangible pathway 700b. Referring now to Figures 6D and 6E, a caregiver may insert a finger or thumb through the second accessibility opening 802b and grasp the first belt 106 and the second fastener component 707b with a first hand. The caregiver's opposing second hand may be used to help stabilize the wearer. For example, the caregiver's opposing second hand may apply a holding or stabilizing force to the wearer at the central region 106c of the first belt 106. The user's first hand may then exert a pulling force Fp on the second grip region 801b of the first belt 106 outward away from the wearer to initiate a tearing of the first belt 106 along the second frangible pathway 700b, such as shown in Figure 6E.

With continued reference to Figure 6E, a pulling force Fp (generally represented by an arrow) is applied to the second grip region 801b in a direction generally toward the second end region 106b of the first belt 106 and/or outward away from the first belt 106. As the pulling force Fp is applied, a first tear line 705a and a second tear line 705b may simultaneously propagate along the first tear zone 813a and the second tear zone 813b, respectively. The first tear line 705a may propagate from the second accessibility opening 802b along the first tear zone 813a of the second frangible pathway 700b in longitudinal and lateral directions partially through and adjacent to the second fastener component 707b and then in a direction D1 that is generally laterally and longitudinally outward from the central region 106c of the first belt 106 and toward the second distal terminus 808b in the second end region 106b of the first belt 106. Simultaneously, the second tear line 705b may propagate from the second accessibility opening 802b in longitudinal and lateral directions partially through and adjacent to the second fastener component 707b along the second tear zone 813b of the second frangible pathway 700b in a direction D2 that is generally laterally outward and longitudinally inward from the central region 106c of the first belt 106 and toward the second proximal terminus 810b in the second end region 106b of the first belt 106.

**In** some configurations, the first tear line 705a may propagate from the second accessibility opening 802b along the first initial tear zone 815a of the second frangible pathway 700b to the first transition zone 817a. From the first transition zone 817a, the first tear line 705a may then propagate along the first secondary tear zone 819a to the second distal terminus 808b. **In** addition, the second tear line 705b may propagate from the second accessibility opening 802b along the second initial tear zone 815b of the second frangible pathway 700b to the second transition zone 817b. From the second transition zone 817b, the second tear line 705b may then propagate along the second secondary tear zone 819b to the second proximal terminus 810b. As discussed in more detail below, the second frangible pathway 700b may be configured such that the first tear line 705a and the second tear line 705b may reach second distal terminus 808b and the second proximal terminus 810b, respectively, at the same time or about the same time.

As shown in Figure 6F, the first belt 106 may be separable along the second frangible pathway 700b to define a second belt zone 832 and a third belt zone 833. For example, the second belt zone 832 may be formed once the first tear line 705a propagates through the second distal terminus 808b and the second tear line 705b propagates through to the second proximal terminus 810b, the second belt zone 832 may be formed. As shown in Figure 6F, a first edge 832a of the second belt zone 832 is formed by tearing the second frangible pathway 700b. In addition, a second edge 833b of the third belt zone 833 is also formed by tearing the second frangible pathway 700b. The second belt zone 832 may extend from the first edge 832a of the first and second tear lines 705a, 705b to the second side seam 180 or the second longitudinal side edge 111b of the first belt 106. In addition, the second belt zone 832 may include the second fastener component 707b. The third belt zone 833 may extend laterally between the first edge 833a and the second edge 833b and may remain connected with the chassis 102.

Although the tearing process is described above with reference to Figures 6A-6F as tearing the first belt 106 along the first frangible pathway 700a before tearing the first belt along the second frangible pathway 700b, it is to be appreciated that the tearing of first belt 106 along the frangible pathways 700 may occur in various different orders and in different manners. For example, the first belt 106 may be torn along second frangible pathway 700b to define the second belt zone 832 before tearing the first belt 106 along the first frangible pathway 700a to define the first belt zone 831. In another example, the first belt 106 may be torn simultaneously along the first frangible pathway 700a and the second frangible pathway 700b to define the first belt zone 831, the second belt zone 832, and the third belt zone 833.

Once the first belt 106 is torn along the frangible pathways 700 to define the first belt zone 831, the second belt zone 832, and the third belt zone 833, the diaper pant 100P may be removed from a wearer in a manner similar to that of a conventional taped diaper. After being removed from a wearer, the diaper pant 100P may be placed in a disposal configuration.

As described above, frangible pathways 700 may be configured such that the first tear line 705a and the second tear line 705b may propagate to reach the distal terminus 808 and the proximal terminus 810, respectively, simultaneously or approximately simultaneously. Such simultaneous tear propagation and tear termination may help provide a caregiver with a convenient and confident diaper pant removal experience. As mentioned above, on occasions when one of the first tear line 705a or the second tear line propagates through the distal terminus 808 or the proximal terminus 810, respectively, significantly in advance of the other tear line, the pulling force needed to complete the tearing may be reduced. In turn, the reduction of force may cause the caregiver to believe the tearing is completed and to stop the tearing process before completion. As such, the caregiver may need to initiate a second tearing motion to complete the tearing process.

As discussed above, the frangible pathways 700 may comprise individual lines of weakness 704. In some configurations, a sum of the lengths of the lines of weakness 704 of the first tear zone 813a may be equal to or substantially equal to a sum of the lengths of the lines of weakness 704 of the second tear zone 813b. In some configurations, a sum of the lengths of the lines of weakness 704 of the first tear zone 813a may be different from a sum of the lengths of the lines of weakness 704 of the second tear zone 813b. The sum of the lengths of the lines of weakness 704 of the first tear zone 813a may be different from a sum of the lengths of the lines of weakness 704 of the second tear zone 813b by from about 1% to about 10%.

As discussed above, frangible pathways 700 may be configured with first and second tear zones 813a, 813b wherein first and second tear lines 705a, 705b propagate through the distal terminus 808 and the proximal terminus 810, respectively. In some configurations, frangible pathways 700 may be configured with first and second tear zones 813a, 813b wherein the first tear line 705a or the second tear line 705b may propagate through the distal terminus 808 or the proximal terminus 810, respectively, before the other.

It is to be appreciated that frangible pathways 700 may be configured with an accessibility opening 802, a first tear zone 813a, and a second tear zone 813b arranged such that the lengths of the first and second tear zones may not be equal or substantially equal, such as shown for example in Figures 7A, 7B, and 7B1. For example, as discussed below in more detail, the frangible pathways illustrated in Figures 7A and 7B may be configured to be operated with a two step tearing process that comprises a first step of tearing the belt along first and second initial tear zones 815a, 815b to the first and second transition zones 817a, 817b, and then subsequently and simultaneously tearing first and second secondary tear zones 819a, 819b from the first and second transition zones 817a, 817b to the distal terminus 808 and the proximal terminus 810, respectively.

Referring now to Figures 7A and 7B, when removing a diaper pant 100P from a wearer, a user may grab the first belt 106 in the grip region 801 by inserting one or more fingers and/or a thumb through the accessibility opening 802 to grasp a portion of the first belt106 and fastener component 707. For example, with reference to Figures 7B and 7C, a caregiver may insert a finger or thumb through the first accessibility opening 802a and grasp the first belt 106 and the first fastener component 707a with a first hand. The caregiver's opposing second hand may be used to help stabilize the wearer, as discussed above. The user's first hand may then exert an initial tear force Fit (generally represented by a curved arrow) on the first grip region 801a of the first belt 106 outward away from the wearer to initiate a tearing of the first belt 106 along the first initial tear zone 815a and the second initial tear zone 815b, such as shown in Figure 7C. As the initial tear force Fit is applied, a first tear line 705a may propagate from the accessibility opening 802 along the first initial tear zone 815a in longitudinal and lateral directions around the first fastener component 707a and then to the first transition zone 817a, such as shown in Figures 7C and 7D. In addition, a second tear line 705b may propagate in a lateral direction from the accessibility opening 802 along the second initial tear zone 815b to the second transition zone 817b.

With reference to Figures 7D and 7E, once the first initial tear zone 815a and the second initial tear zone 815b are completely separated, a pulling force Fp (generally represented by an arrow) may be applied to the first grip region 801a in a direction generally toward the first end region 106a of the first belt 106 and/or outward away from the first belt 106 and the wearer. As the pulling force Fp is applied, the first tear line 705a and the second tear line 705b may simultaneously propagate along the first secondary tear zone 819a and the second secondary tear zone 819b, respectively. The first tear line 705a may propagate from the first transition zone 817a along the first secondary tear zone 819a of the first frangible pathway 700a in a direction D1 that is generally laterally and longitudinally outward from the central region 106c of the first belt 106 and toward the first distal terminus 808a in the first end region 106a of the first belt 106. Simultaneously, the second tear line 705b may propagate from the second transition zone 817b along the second secondary tear zone 819b of the first frangible pathway 700a in a direction D2 that is generally laterally outward and longitudinally inward from the central region 106c of the first belt 106 and toward the first proximal terminus 810a in the first end region 106a of the first belt 106. The second frangible pathway 700b may be configured such that the first tear line 705a and the second tear line 705b may reach second distal terminus 808b and the second proximal terminus 810b, respectively, at the same time or about the same time.

It is to be appreciated that the frangible pathways 700 may be configured in various different ways with various zones having different lengths. It is also to be appreciated that the belt may comprise one or more substrates and the frangible pathway may extend through each of the one or more substrates, and the frangible pathway extending through two or more substrates forming the belt may be aligned, such that the frangible pathway of the first substrate is aligned with the frangible pathway of the second substrate. Alternatively, the frangible pathways extending through two or more substrates forming the belt may not be aligned or is partially aligned, such that at least a portion of the frangible pathway of the first substrate is offset with the frangible pathway of the second substrate.

Figures 8A-8D illustrate various example embodiments of a diaper pant 100P having different frangible pathways 700. As illustrated in Figure 8A, the frangible pathway 700 may comprise a first frangible pathway 700a and a second frangible pathway 700b. Each of the first frangible pathway 700a and the second frangible pathway 700b may comprises a first tear zone 813a and a second tear zone 813b. A transition zone 817a, 817b may join the first tear zone 813a and the second tear zone 813b. The Each of the first tear zone 813a and the second tear zone 813b may form a pathway angle β with respect to the lateral centerline of the belt 126a. The pathway angle β of the first tear zone 813a and the pathway angle β of the second tear zone 813b may be the same or different. As illustrated in Figure 8A, the pathway angle β of the first tear zone 813a is less than the pathway angle β of the second tear zone 813b. Further, the length of the first tear zone 813a may be greater than or less than the length the second tear zone 813b. Alternatively, the length of the first tear zone 813a may be substantially the same as the length of the second tear zone 813b. The diaper pant 100P may also comprise a fastener component 707, as previously discussed. The fastener component 707 may be positioned such that a portion of the frangible pathway 700 surrounds a portion of the fastener component 707, and the fastener component 707 may be positioned such that the distance from the centroid of the fastener component 707 to the outer edge 107a of the belt is not equal to the distance from the centroid of the fastener component 707 to the inner edge 107b of the belt, where the distance is the shortest straight-line distance between these features.

As illustrated in Figure 8B, the frangible pathway 700 may comprise a single tear zone 813. The tear zone 813 may extend between the outer edge 107a and the inner edge 107b of the belt. The frangible pathway 700, as illustrated in Figure 8B, may have a pathway angle β with respect to the lateral centerline 126a of the belt. Each of the first frangible pathway 700a and the second frangible pathway 700b may form a pathway angle with respect to the lateral centerline 126a, and each of these pathway angles may be the same or different. The diaper pant 100P may also comprise a fastener component 707. The fastener component 707 may be positioned adjacent to the frangible pathway 700. The frangible pathway 700 may intersect a portion of the fastener component 707. The fastener component 707 may be positioned such that the fastener component 707 does not overlap the lateral centerline 126a of the belt. The centroid of the fastener component 707 may be positioned such that the distance between the centroid of the fastener component 707 is closer to either the first the outer edge 107a of the belt or the inner edge 107b of the belt, where the distance is the shortest straight-line distance between these features.

As illustrated in Figure 8C, the frangible pathway 700 may comprise a first frangible pathway 700a and a second frangible pathway 700b. Each of the first frangible pathway 700a and the second frangible pathway 700b may comprises a first tear zone 813a and a second tear zone 813b. A transition zone 817 may join the first tear zone 813a and the second tear zone 813b. The Each of the first tear zone 813a and the second tear zone 813b may form a pathway angle β with respect to the later centerline of the belt 126a. The pathway angle β of the first tear zone 813a and the pathway angle β of the second tear zone 813b may be different. As illustrated in Figure 8C, the pathway angle β of the first tear zone 813a is greater than the pathway angle β of the second tear zone 813b. The pathway angle β of the first tear zone 813a is about 90 degrees from the lateral centerline of the belt and the pathway angle β of the second tear zone 813b is less than 90 degrees from the lateral centerline of the belt. Further, the length of the first tear zone 813a may be less than the second tear zone 813b. The diaper pant 100P may also comprise a fastener component 707, as previously discussed. The fastener component 707 may be positioned such that a portion of the frangible pathway 700 intersects the fastener component 707, and the fastener component 707 may be positioned such that the distance from the centroid of the fastener component 707 to the outer edge 107a of the belt is not equal to the distance from the centroid of the fastener component 707 to the inner edge 107b of the belt, where the distance is the shortest straight-line distance between these features.

Further, as illustrated in each of Figures 8A-8C, the diaper pant 100P may comprise an accessibility opening 802. The accessibility opening 802 may be positioned at an angle with respect to the lateral centerline of the belt. The accessibility opening 802 may be positioned adjacent to the fastener component or may be disposed on at least a portion of the fastener component.

As previously discussed, with reference to Figures 6C, 7D, 7E, and 8A-8C the pulling force Fp may be applied in a direction generally toward the first end region 106a of the first belt 106 and/or outward away from the first belt 106 and the wearer. As the force Fp is applied, a first tear line 705a and a second tear line 705b may propagate along the first tear zone 813a and the second tear zone 813b, respectively. Alternatively, as the force Fp, is applied, a tear line may propagate along the tear zone 813. However, due to the direction in which the pulling force is executed, the tear could propagate in an unintended tear direction UTD, such as indicated in Figure 8D. An unintended tear direction UTD is any tearing of the belt other than along the frangible pathway when the user is attempting to tear along the frangible pathway. It is to be appreciated that this tearing may occur in one or more layers of the belt. If the tear were to propagate in the unintended tear direction UTD rather than along the frangible pathway, this would adversely interfere with the removal of the absorbent article. For example, if a portion of the tear extends into a portion of the belt rather than along the frangible pathway, the caregiver may sense or feel a change in force, and thus, may stop the tearing motion, assuming the tear along the tear zones is complete. Upon realizing that the tear is not complete, the caregiver may have to re-grip the belt and complete the tear of the incompletely torn frangible pathway in a separate step. Such starting, stopping, and restarting the tearing process may require the user utilize both hands and may require additional time to complete the removal of the article from a wearer. In another example, if the tear propagates in the unintended tear direction, the caregiver may not be able to complete the tear to remove the absorbent article in a manner similar to a taped-type absorbent article and, alternatively, would be forced to remove the absorbent article over the legs of the wearer causing the contents of the absorbent article to be unsecured and next to the skin of the wearer during removal. Thus, it is important to design the frangible pathway such that the tear does not propagate along an unintended tear direction UTD, but rather along the frangible pathway.

Referring to Figures 9A-9C, the frangible pathway 700 may extend in a first pathway direction FPD and the force to tear FT may be in a second direction. The first pathway direction FPD may be different than the force to tear FT in the second direction. Alternatively, the first pathway direction FPD may be the same as the force to tear FT in the second direction. When the first pathway direction FPD is different than the force to tear FT in the second direction, it may be more likely that the tear wants to propagate in an unintended tear direction UTD. As previously stated, the substrate, also referred to herein as a material, having the frangible pathway may be weaker in one or more directions due to the characteristics of the material. For example, the frangible pathway may extend through at least a portion of a nonwoven material. The nonwoven material may include a plurality of fibers oriented in a fiber orientation direction and having a bond pattern that holds together the fibers to form the nonwoven. When the force to tear FT the frangible pathway includes a force component that is in a direction parallel or substantially parallel to the weakest direction of the material through which the frangible pathway extends, the pathway tear strength needs to be sufficiently less than the material tear strength to prevent tearing in an unintended tear direction. Figures 9A-9C illustrate various examples of the frangible pathway 700 having a frangible pathway direction and a force to tear FT having a direction, and the force to tear FT having a horizontal force to tear component FT_{H} and a vertical force to tear component FT_{V}. Due to the force to tear FT having directional components that are substantially parallel to the weakest material direction or weakest substrate direction, the tear may be more susceptible to propagate outside of the frangible pathway.

To avoid tearing in the belt and/or one or more layers of the substrate in an unintended tear direction, it has been found that the tear strength of each substrate should be greater than the tear strength of the frangible pathway. The difference between the tear strength of the substrate and the tear strength of the frangible pathway are such that the tear remains on the frangible pathway as the user tears the belt along the frangible pathway.

The characteristics of the belt determine the material tear strength. The belt 106, 108 may comprise one or more substrates, such as a first substrate 162, and/or second substrate 164, and may be constructed from various materials. As previously discussed, the first and/or second belts may include a first substrate 162, and/or second substrate 164 that may be manufactured from materials such as plastic films; apertured plastic films; woven or nonwoven webs of natural materials (e.g., wood or cotton fibers), synthetic fibers (e.g., polyolefins, polyamides, polyester, polyethylene, or polypropylene fibers) or a combination of natural and/or synthetic fibers; or coated woven or nonwoven webs. In some configurations, the first and/or second belts may include a first substrate 162, and/or second substrate 164 comprising a nonwoven web of synthetic fibers and may include a stretchable nonwoven. The type of materials used for the one or more substrates of the belt influences the material tear strength. It is also to be appreciated that a belt may comprise a single substrate or a belt may comprise two or more substrates.

Further, nonwoven belts are manufactured using various techniques in which, generally, fibers are laid down onto a carrier and one or more bond patterns may be used to secure of the fibers of the substrate. The direction in which the carrier is moving, the machine direction MD, onto which the fibers are laid down is the direction in which the majority of the fibers of the substrate are oriented, referred to herein as a fiber orientation direction. Accordingly, the fiber orientation direction is substantially parallel to the machine direction MD. The shape and frequency of bonds throughout the substrate also influences the strength of the belt. Thus, the belt may be stronger along certain axes given the fiber orientation direction and the characteristics of the bond pattern. Example bond patterns in various configurations are described in U.S. Patent Publication Nos. 2017/0000663; 2014/0088535; 2016/0324698; and 2016/0101003; and U.S. Patent Nos. 9,993,369; 10,028,866; and 9,408,761. Generally, the larger the open areas, areas devoid of bonds, the lower the material strength. The substrate, which may be an outer garment facing surface, an inner wearer facing surface, or an intermediate layer of the belt, may include a plurality of fibers having a denier of less than about 1.5, or less than about 1.3, or less than about 1.2, or less than about 1, specifically reciting all 0.1 increments within the above-recited range and all ranges formed therein or thereby.

Further still, the belt and, individually, the one or more substrates forming the belt may have a basis weight. Generally, the higher the basis weight of the substrate and/or the belt, the greater the tear strength of the belt and/or substrate. Each substrate layer may have a basis weight that provides sufficient tear strength. The basis weight of the one or more substrates may be optimized so that the desired tear strength is reached with the desired softness and air permeability. Stated another way, the higher the basis weight of the one or more substrates, the greater the stiffness of the one or more substrates. Thus, the basis weight may be selected such that it provides the necessary strength to the substrate while being soft and comfortable against the wearer's skin during use. The basis weight of the belt may be less than about 60 gsm, or less than about 50 gsm, or less than about 45 gsm, or less than about 40 gsm, or less than about 35 gsm, or less than about 30 gsm, or less than about 25 gsm, or less than about 20 gsm, specifically reciting all 0.1 gsm increments within the above-recited range and all ranges formed therein or thereby. The basis weight of the belt is the sum of the basis weight for each substrate layer used to form the belt, which may be one or more substrates. The substrate may have a basis weight of less than about 30 gsm, or less than about 25 gsm, or less than about 20 gsm, or less than about 15 gsm, or less than about 10 gsm, or less than about 8 gsm, or from about 6 gsm to about 30 gsm, or from about 8 gsm or about 20 gsm, or from about 8 gsm to about 11 gsm, specifically reciting all 0.1 gsm increments within the above-recited range and all ranges formed therein or thereby.

It is to be appreciated that the substrate may include a softness enhancer additive to increase the softness of the substrate. Thus, a higher basis weight material may be used to achieve greater strength and a softness enhancer additive may be used to impart softness to the substrate. The softness enhancer additive may be added to the composition in neat form, diluted, and/or as a masterbatch in, for example, polyolefin polymers such as polypropylene, polystyrene, low density polyethylene, high density polyethylene, or propylene-alpha-olefin copolymers. A composition suitable to make fibers as described herein contains one or more softness enhancer additive, which can be present in an amount of between 0.01% to 10%, or between 0.05% to 5%, or between 0.3% to 1%, or between 0.01% to 1%, or between 0.2% to 0.5% by weight of the fibers, specifically reciting all 0.1% increments within the above recited range and all ranges formed therein or thereby. Example softness enhancer additives in various configurations are disclosed in U.S. Patent No. 9,993,369, The softness enhancer additive may be on a single side, such as the garment facing surface or the wearer facing surface of the belt. The softness enhance additive may be present in different amounts on each of the garment facing surface of the belt and the wearer facing surface of the belt.

At least a portion of the frangible pathway 700 may be at a pathway angle β with respect to lateral centerline of the elastic belt 126a, 126b through which the frangible pathway may extend. The frangible pathway angle β may be less than 90 degrees, or less than 85 degrees, or less than 75 degrees, or less than 65 degrees, or less than 45 degrees, or less than 35 degrees, or less than 25 degrees, or less than 15 degrees, specifically reciting all 0.1 degree increments within the above-recited range and all ranges formed therein or thereby. The frangible pathway angle β may be from 0 degrees to 85 degrees, or from about 15 degrees to about 75 degrees, or from about 30 degrees to about 60 degrees, or from about 30 degrees to about 45 degrees, or from about 0 degrees to about 45 degrees, specifically reciting all 0.1 degree increments within the above-recited range and all ranges formed therein or thereby. For example, as illustrated in Figures 8A-8D, the frangible pathway 700 may include one or more frangible pathways, such as a first frangible pathway 700a and a second frangible pathway 700b. Each of the first frangible pathway 700a and the second frangible pathway 700b may from a pathway angle β with respect to the lateral centerline of the first elastic belt 126a. Figures 9A-9C illustrate examples of frangible pathways 700 having a frangible pathway angle β with respect to the lateral centerline 126a. It is to be appreciated that the frangible pathway angle may apply to the entire frangible pathway or one or more portions of the frangible pathway may have a frangible pathway angle as described herein.

Additionally or alternatively, at least a portion of the frangible pathway700 may be at a pathway angle α with respect to fiber orientation direction 720 of the substrate through which the frangible pathway extends. The fiber orientation direction 720, as previously discussed, is the direction in which the majority of the fibers are oriented, which is substantially parallel to the machine direction in which the fibers are laid down during the production process. The pathway angle α may be less than 90 degrees, or less than 80 degrees, or less than 75 degrees, or less than 65 degrees, or less than 45 degrees, or less than 35 degrees, or less than 25 degrees, or less than 15 degrees, specifically reciting all 0.1 degree increments within the above-recited range and all ranges formed therein or thereby. The frangible pathway angle α may be from 0 degrees to 85 degrees, or from about 15 degrees to about 75 degrees, or from about 30 degrees to about 60 degrees, or from about 30 degrees to about 45 degrees, or from about 0 degrees to about 45 degrees, specifically reciting all 0.1 degree increments within the above-recited range and all ranges formed therein or thereby. For example, Figures 9D-9E illustrate examples of frangible pathways 700 having a frangible pathway angle α with respect to the fiber orientation direction 720. The fiber orientation direction may be substantially parallel to the lateral centerline 126a of the belt or at an angle with respect to the lateral centerline of the belt. Generally, the greater the frangible pathway angle α with respect to the fiber orientation direction 720, the more likely the tear may propagate in the unintended tear direction and, thus, the greater the difference between the pathway tear strength and the material tear strength to prevent the tear from propagating in the unintended tear direction.

It has been found that to prevent tearing in an unintended tear direction UTD, the tear strength of the one or more substrates forming the belt, referred to herein as the material tear strength, must be greater than the tear strength of the frangible pathway, referred to herein as the pathway tear strength. Stated another way, the pathway tear strength must be less than the material tear strength. More specifically, the difference between the pathway tear strength and the material tear strength may be greater than about 2N, or greater than about 3 N, or greater than about 5 N, or greater than about 8 N, or greater than about 10 N, or greater than about 15 N, or greater than about 20 N, or from about 4 N to about 30 N, or from about 4 N to about 25 N, or from about 5 N to about 20 N, or from about 10 N to about 20 N, or from about 15 N to about 20 N, according to the Trapezoid Tear Strength test method disclosed herein, specifically reciting all 0.1 N increments within the above-recited range and all ranges formed therein or thereby. Generally, the larger the difference between the pathway tear strength and the material tear strength the more unlikely the tear will propagate in an unintended tear direction. However, strength must be balanced with softness and other material properties so that the substrate can be used comfortably against the skin of the wearer. Each of the material tear strength and the pathway tear strength may be such that the belt maintains proper position of the absorbent article during use, the pathway tear strength maintains closure of the belt during use, and the frangible pathway is easily opened during removal of the diaper pant. The pathway tear strength may be greater than about 2 N, or greater than about 3 N, or greater than about 5 N, or greater than about 10 N, or greater than about 15 N, or from about 2 N to about 20 N, or from about 2 N to about 16 N, or from about 5 N to about 10 N, or from about 6 N to about 8 N, according to Trapezoid Tear Strength test method, specifically reciting all 0.1 N increments within the above-recited range and all ranges formed therein or thereby. The material tear strength may be greater than about 2 N, or greater than about 4 N, or greater than about 6 N, or greater than about 8 N, or greater than about 12 N, or greater than about 20 N, or greater than about 25 N, or greater than about 30 N, or from about 4 N to about 40 N, or from about 7 N to about 25 N, or from about 8 N to about 20 N, or from about 8 N to about 15 N, or from about 8 N to about 10 N, according to Trapezoid Tear Strength test method, specifically reciting all 0.1 N increments within the above-recited range and all ranges formed therein or thereby. It is to be appreciated that the frangible pathway may include one or more tear zones, as previously discussed. Figure 10 illustrates graphically the difference between the material tear strength and the pathway tear strength that each may be calculated using the Trapezoid Tear Strength test method. It is also to be appreciated that the belt may include one or more substrate layers and one or more elastics. The elastics are severed in the region of the frangible pathway as to not interfere with tearing along the frangible pathway.

The difference between the material tear strength and the pathway tear strength may also be described with reference to the basis weight of the one or more materials used in the belt. Generally, the higher the basis weight of the material, the stronger the material. Higher basis weight materials may result in a greater difference between the material tear strength and the pathway tear strength. However, higher basis weight materials also, generally, have increased material stiffness, decreased air permeability, and require greater amounts of raw material for manufacture. Thus, by normalizing the material tear strength and the pathway tear strength by basis weight, it can be determined which materials or substrates may be strong enough to deter or prevent tearing in an unintended tear direction while balancing the properties of the material, such as softness, air permeability, and raw material usage. It has been found that the following parameters result in a substrate comprising a frangible pathway that tears along the frangible pathway and minimizes the likelihood that the tear will propagate in an unintended tear direction. The material tear strength divided by the basis weight of the substrate or the belt, referred to herein as the material basis weight, is greater than 0.2 N/gsm, or greater than about 0.3 N/gsm, or greater than about 0.4 N/gsm, or from about 0.2 N/gsm to about 0.4 N/gsm, or from about 0.3 N/gsm to about 0.5 N/gsm, specifically reciting all 0.01 N/gsm increments within the above-recited range and all ranges formed therein or thereby. It is to be appreciated that this includes each substrate layer individually or the belt, including all substrate layers. It is also to be appreciated that each substrate layer may have different basis weights and thus, different values of the material tear strength divided by the basis weight. Further, it has been found that the pathway tear strength divided by the material basis weight is greater than about 0.05 N/gsm to less than about 0.15 N/gsm, to achieve tearing along the frangible pathway for a given material. Further still, the difference between the material tear strength and the pathway tear strength is referred to herein as a tear strength delta. The tear strength delta divided by the basis weight of the material should be greater than 0.2 N/gsm, or greater than about 0.3 N/gsm, or from about 0.2 N/gsm to about 0.4 N/gsm, specifically reciting all 0.01 N/gsm increments within the above-recited range and all ranges formed therein or thereby, to have a substrate that is strong enough to deter tearing in an unintended tear direction and is consumer preferred, which may be due, in part, to the softness or breathability.

In addition to having sufficient tear strength, the belt may also be softness so that the diaper pant is comfortable for wearing. The belt may comprise one or more surfaces which comprise an Average TS7 value of about 10 dB V² rms or less, or about 7 dB V² rms or less, or about 5 dB V² rms or less, or from about 1 dB V² rms to about 10 dB V² rms, or from about 2 dB V² rms to about 8 dB V² rms according to the Softness Test Method herein, specifically reciting all 0.1 dB V² rms increments within the above-recited range and all ranges formed therein or thereby. Additionally or alternatively, the belt may comprise one or more surfaces with an Average TS750 value of about 75 dB V² rms or less, or about 50 dB V² rms or less, or about 25 dB V² rms or less, or about 15 dB V² rms or less, or from about 10 dB V² rms to about 100 dB V² rms, or from about 10 dB V² rms to about 80 dB V² rms, or from about 15 dB V² rms to about 75 dB V² rms according to the Softness Test Method herein, specifically reciting all 0.1 dB V² rms increments within the above-recited range and all ranges formed therein or thereby. A substrate of the belt may comprise one or more surfaces with an Average TS750 value of about 11 dB V2 rms or less, or about 10 dB V² rms or less, or about 5 dB V² rms or less, or from about 1 dB V² rms to about 11 dB V² rms, or from about 1.5 dB V² rms to about 6 dB V² rms, or from about 2 dB V² rms to about 4 dB V² rms according to the Softness Test Method herein, reciting for each range every 0.1 dB V² rms increment therein. Lower TS7 and TS750 values indicate greater softness, which is highly desirable in absorbent articles. Consumers might find absorbent articles with high TS7 and TS750 values uncomfortable and/or scratchy or otherwise undesirable.

The following are several example embodiments. Data for Examples 1-10 are included in Table 1 below. Each of Examples 1-10 include a substrate having a frangible pathway at a pathway angle of 35 degrees with respect to the machine direction MD or fiber orientation direction of the substrate. Each substrate of Examples 1-10 has a basis weight and has certain material properties, as indicated in Table 1. The pathway tear strength and the material tear strength of each of the substrate and the frangible pathway was generated using the Trapezoid Tear Strength test method as disclosed herein. Examples 1-10 include a bond pattern as illustrated in one of Figures 11A-11B and 12A-12B.

As discussed herein, the material tear strength divided by basis weight, the frangible pathway tear strength divided by basis weight, and the delta, which is the material tear strength minus frangible pathway tear strength, divided by basis weight may be used to determine which substrates have sufficient strength while balancing other properties of the substrate such as stiffness, air permeability, and raw material usage. By increasing basis weight to a relatively high level, product softness, skin comfort, and cost are negatively impacted. By normalizing the difference between material tear strength and pathway tear strength to basis weight, a material having the desired tear strength so that the substrate is unlikely to tear in an unintended trear direction and is sufficiently soft and breathable to be used in a pant diaper 100P.

Example 1 includes a substrate having a relatively high basis weight of 30 gsm, which indicates a strong material. The material tear strength is sufficiently greater than the pathway tear strength, so it is unlikely this substrate would tear in an unintended tear direction. However, Example 1 has a delta tear strength, which is the difference between the material tear strength and the pathway tear strength, divided by basis weight of less than 0.185 N/gms and, thus, this substrate is less than 0.2 N/gsm and is not preferred. It is likely this substrate would be too stiff, be costly to produce, and would not be consumer preferred when contacting the skin. Each of Examples 2-7, have a material tear strength that is sufficiently greater than the pathway tear strength (delta greater than 2N), so it is unlikely these substrates would tear in an unintended tear direction. Further, Examples 2-7 each have a tear strength delta divided by basis weight of about 0.2 N/gsm indicating that these would be materials that would not tear in an unintended tear direction and would be materials that are relatively cost effective, soft, and consumer preferred for contact with the skin. Further still, Examples 2-7 each have a material tear strength divided by basis weight of about 0.3 N/gsm, which indicates a substrate having sufficient strength based on the basis weight of the material. Examples 8 and 9 include substrates having a material tear strength that is about 2 N greater than the pathway tear strength. However, each of Examples 8 and 9 have a material tear strength divided by basis weight that is less than 0.3 N/gm and a tear strength delta that is less than 0.2 N/gsm, which indicates that these substrates do not have a sufficient tear strength when normalized by basis weight and would likely have tearing in an unintended tear direction. Example 10 includes a substrate having a relatively low basis weight and the difference between the material tear strength and the pathway tear strength is less than 2 N. The substrate of Example 10 would likely have tearing in an unintended tear direction due to the minimal difference between the material and pathway tear strengths. Further, the substrate of Example 10 does not have sufficient strength due to the relatively low basis weight.

In summary, those substrates having a difference in material tear strength and pathway tear strength of at least 2 N and, more preferably, at least 3 N, and a material tear strength divided by basis weight or at least 0.2 N/gsm and, more preferably at least 0.3 N/gsm, and a delta divided by basis weight of at least 0.2 N/gsm and, more preferably at least 0.3 N/gsm have sufficient strength to avoid tearing in an unintended tear direction while the user is tearing along the frangible pathway to remove or examine the contents of the diaper pant and has desired properties, such as softness, breathability, and conscious raw material usage.

**Table 1.**

| | **Material Process** | **Bond Pattern** | **Pathway Angle (degrees)** | **Basis Weight (gsm)** | **Material Tear Strength (N)** | **Frangible Pathway Tear Strength (N)** | **Delta: Material Tear Strength - Frangible Pathway Tear Strength (N)** | **Material Tear Strength / Basis Weight (N/gsm)** | **Frangible Pathway Tear Strength/ Basis Weight (N/gsm)** | **Delta / Basis Weight (N/gsm)** |
|---|---|---|---|---|---|---|---|---|---|---|
| Example 1 | Hydrojetted spunbond with softblend | A-BP | 35 | 30 | 8.7 | 3.2 | 5.6 | 0.291 | 0.11 | 0.185 |
| Example 2 | Polypropylene Spunbond | A-BP | 35 | 25 | 8.3 | 2.3 | 6 | 0.332 | 0.09 | 0.24 |
| Example 3 | Polypropylene Spunbond | A-BP | 35 | 19 | 7 | 2 | 5 | 0.368 | 0.11 | 0.262 |
| Example 4 | Polypropylene Spunbond | A-BP | 35 | 17 | 5.5 | 1.5 | 4 | 0.324 | 0.09 | 0.235 |
| Example 5 | Bi-component PP/PE Spunbond | B-BP | 35 | 17 | 6.2 | 1.6 | 4.6 | 0.365 | 0.09 | 0.271 |
| Example 6 | Spunbond-Meltblown-Spunbond (SMS) | C-BP | 35 | 16 | 5.4 | 1.7 | 3.7 | 0.338 | 0.11 | 0.231 |
| Example 7 | Spunbond-Meltblown-Spunbond (SMS) | C-BP | 35 | 15 | 4.8 | 1.5 | 3.3 | 0.32 | 0.1 | 0.22 |
| Example 8 | Spunbond with Softblend | B-BP | 35 | 15 | 3.4 | 1.2 | 2.1 | 0.224 | 0.08 | 0.143 |
| Example 9 | Bi-component PP/PE Spunbond | D-BP | 35 | 15 | 3.7 | 1.5 | 2.2 | 0.247 | 0.1 | 0.147 |
| Example 10 | Spunbond-Meltblown-Spunbond (SMS) | B-BP | 35 | 8 | 2 | 0.8 | 1.2 | 0.25 | 0.1 | 0.15 |

With reference to various aspects of the Figures described above, it is to be appreciated that grip regions 801 and accessibility openings 802 may be located in various positions in the first end region 106a, the second end region 106b, and/or the central region 106c of the first belt 106. Grip regions 801 and accessibility openings 802 may be positioned between the first longitudinal side edge 111a, the second longitudinal side edge 111b, the outer edge 107a, and the inner edge 107b of the first belt 106. For example, the first accessibility opening 802a and/or the second accessibility 802b may be entirely laterally positioned between the first longitudinal edge 128 and the second longitudinal edge 130 of the chassis 102. In some configurations, the first accessibility opening 802a may be positioned laterally between the first longitudinal side edge 128 of the chassis 102 and the first longitudinal side edge 111a of the first belt 106 and/or first side seam 178. In some configurations, the second accessibility opening 802b may be positioned laterally between the second longitudinal side edge 130 of the chassis 102 and the second longitudinal side edge 111b of the first belt 106 and/or second side seam 180. In some configurations, the first accessibility opening 802a and/or the second accessibility opening 802b may be positioned longitudinally between the first lateral edge 144 of the chassis 102 and the inner edge 107b of the first belt 106 and/or may be positioned longitudinally between the first lateral edge 144 of the chassis 102 and the outer edge 107a of the first belt 106. In some configurations, the first accessibility opening 802a may extend across the first longitudinal edge 128 and/or the first lateral edge 144 of the chassis 102, and/or the second accessibility opening 802b may extend across the second longitudinal edge 130 and/or the first lateral edge 144 of the chassis 102.

It is also be appreciated that accessibility openings 802 may be located in various positions relative to fastener components 707. For example, in some configurations, the accessibility opening 802 may be positioned longitudinally between the fastener component 707 and the outer edge 107a of the first belt 106. In some configurations, the accessibility opening 802 may be positioned longitudinally between the fastener component 707 and the inner edge 107b of the first belt 106. In some configurations, the accessibility opening 802 may be positioned laterally inboard of the fastener component 707. It is also to be appreciated that more than one accessibility opening 802 may be located adjacent a fastener component 707. As discussed in more detail below, the accessibility opening 802 may also be configured to extend partially or entirely through a fastener component 707 and may divide a fastener component 707 into two or more parts.

As mentioned above, the accessibility opening 802 may comprise slits and/or openings in the first belt 106 and may be curved and/or straight. It is to be appreciated that the accessibility openings 802 may also be oriented in various ways. For example, the accessibility opening 802 may be generally oriented perpendicularly relative to the outer edge 107a and/or the inner edge 107b of the first belt 106. In some configurations, the accessibility opening 802 may be generally oriented parallel relative to the outer edge 107a and/or the inner edge 107b of the first belt 106. In some configurations, the accessibility opening 802 may comprise a slit that extends along a line in a lateral direction to define an angle from about 0 degrees to about 45 degrees with respect to the outer edge 107a and/or the inner edge 107b of the first belt 106, specifically reciting all 1 degree increments within the above-recited range and all ranges formed therein or thereby. In some configurations, the accessibility opening 802 may define a length dimension in the range of about 5 mm to about 50 mm, specifically reciting all 0.1 mm increments within the above-recited range and all ranges formed therein or thereby.

As discussed above, the diaper pant 100P may include one or more fastener components 707 adapted to refastenably connect with at least one other component of the diaper pant 100P in a disposal configuration. It is to be appreciated that the fastener components 707 may be configured in various shapes and sizes, and may be located in various positions relative to other components of the diaper pant 100P. The fastener components 707 may comprise a lateral centerline oriented substantially parallel to the lateral centerline 126a of the first elastic belt 106 and/or the lateral centerline 126b of the second elastic belt 108 and/or the lateral centerline 126c of the chassis 102. The fastener components 707 may comprise a longitudinal centerline oriented substantially parallel to the longitudinal centerline 124a of the first elastic belt 106 and/or the longitudinal centerline 124b of the second elastic belt 108 and/or the longitudinal centerline 124c of the chassis 102.

As shown in Figure 8AA1, in some configurations, fastener components 707 may be positioned on and connected with the wearer facing surface 115b of the first elastic belt 106 and/or the second elastic belt 108 in a region where the first elastic belt 106 and/or second elastic belt 108 overlaps the chassis 102. In some configurations, the fastener component 707 may be sandwiched between the second substrate 164 of the first elastic belt 106 or the second elastic belt 108 and the backsheet 136 of the chassis 102. In some configurations, such as shown in Figure 8AA1, the fastener component 707 comprises hooks 715 protruding from a base 717, and the hooks 715 extend from the first belt 106 toward the backsheet 136. The fastener component 707 may be configured as a separate discrete element that may be connected with the wearer facing surface 115b of the first belt 106 in various ways. For example, as shown in Figure 13, adhesive 716 may connect the base 717 of the fastener component 707 with wearer facing surface 115b of the first belt 106. It is to be appreciated that the fastener component 707 may be connected with the first belt 106 by mechanical bonding in addition to or instead of adhesive. It is to be appreciated that the base 717 may be configured in various ways. For example, the base 717 may comprise a thermoplastic film. In some configurations, the base 717 may comprise a laminate with various layers bonded together, such as disclosed for example in U. S. Patent Publication No. 2021/0045931 A1. For example, the base 717 may comprise a thermoplastic film layer bonded with a nonwoven layer. It is to be appreciated that such layers may be bonded together in various ways, such as with adhesive, mechanical bonding, and/or extrusion bonding. In some configurations, the fastener component 707 may be integrally formed from materials of the first belt 106, such as shown for example in Figure 14, or may be integrally formed from materials and attached with the first belt.

A portion of the chassis 102 may overlap the inner wearer facing surface 115b of the first belt 106 to define a chassis overlap region 850. As such, the chassis overlap region 850 may extend laterally between the first longitudinal edge 128 and the second longitudinal edge 130 of the chassis 102 and longitudinally between the first lateral edge 144 of the chassis 102 and the inner edge 107b of the first belt 106. To help prevent contact of the fastener component 707 with a wearer's skin while wearing the diaper pant 100P, the fastener components 707 may be positioned on and connected with the wearer facing surface 115b of the first elastic belt 106 and/or the wearer facing surface 117b of the second elastic belt 108 in the chassis overlap region 850 where the first elastic belt 106 and/or second elastic belt 108 overlaps the chassis 102. For example, the fastener component 707 may be sandwiched between the wearer facing surface 115b of the first belt 106 and the chassis 102. In some configurations, the fastener component 707 may be sandwiched between the second substrate 164 of the first elastic belt 106 or the second elastic belt 108 and the backsheet 136 of the chassis 102. In some configurations, the fastener component 707 may be positioned laterally between the first longitudinal side edge 128 and the second longitudinal side edge 130 of the chassis 102. The fastener component 707 may also be positioned longitudinally between the first lateral edge 144 of the chassis 102 and the inner edge 107b of the first belt 106. The fastener component 707 may be positioned adjacent the frangible pathway 700. The accessibility opening 802, which may be considered part of the frangible pathway 700, may be positioned adjacent the fastener component 707. As such, the frangible pathway 700 may partially surround the fastener component 707. In some configurations, the frangible pathway 700 may extend through the fastener component 700, effectively dividing the fastener component 707 into a first fastener part and a second fastener part. As discussed above, the first fastener part is separated from the second fastener part as the frangible pathway is torn. When completing the tearing operation, the first belt zone 831 and the second belt zone 832 will include first fastener parts, and the third belt zone 833 will include second fastener parts separated from respective first fastener parts during the tearing of frangible pathways 700.

Both the frangible pathway 700 and the accessibility opening 802 may extend through the fastener component, effectively dividing the fastener component 707 into a first fastener part and a second fastener part. The accessibility openings 802 may comprise slits that are generally oriented in a longitudinal direction. In addition, the accessibility opening 802 extends through the fastener component 707 and may be positioned entirely within a perimeter of the fastener component. It is to be appreciated that such slits may be straight and/or curved. In some configurations, a longitudinally extending accessibility opening 802 may define a length dimension in the range of about 10 mm to about 30 mm, specifically reciting all 0.1 mm increments within the above-recited range and all ranges formed therein or thereby. In addition, in some configurations, a longitudinally extending accessibility opening 802 may also be curved to extend laterally in the range of about 2 mm to about 20 mm, specifically reciting all 0.1 mm increments within the above-recited range and all ranges formed therein or thereby.

In another configuration, the diaper pant 100P may comprise one fastener component 707 joined to the wearer facing surface 115b of the first belt 106 in a location overlapping the longitudinal centerline 124c of the chassis 102. The longitudinal centerline of the fastener component 707 may be coincident with, or in proximity of, the longitudinal centerline 124c of the chassis 102. The frangible pathway 700 may divide fastener component 707 into the first fastener component and the second fastener component of substantially similar size and geometry. An accessibility opening 802 may be disposed at, or in proximity of, a longitudinally inboard lateral edge of the fastener component 707. Longitudinally outboard the lateral edges of the fastener component 707, the frangible pathway 700 may extend in longitudinal and lateral directions to the waist edge 121 and inner edge 107b of the first belt 106. A caregiver or wearer may access and grasp the fastener component 707 through the accessibility opening 802 and subsequently separate the frangible pathway 700 into the first and second fastener components.

Components of the absorbent articles described herein may at least partially be comprised of bio-based content as described in U.S. Pat. Appl. No. 2007/0219521A1. For example, the superabsorbent polymer component may be bio-based via their derivation from bio-based acrylic acid. Bio-based acrylic acid and methods of production are further described in U.S. Pat. Appl. Pub. No. 2007/0219521 and U.S. Pat. Nos. 8,703,450; 9,630,901 and 9,822,197. Other components, for example nonwoven and film components, may comprise bio-based polyolefin materials. Bio-based polyolefins are further discussed in U.S. Pat. Appl. Pub. Nos. 2011/0139657, 2011/0139658, 2011/0152812, and 2016/0206774, and U.S. Pat. No. 9,169,366. Example bio-based polyolefins for use in the present disclosure comprise polymers available under the designations SHA7260^{™}, SHE150^{™}, or SGM9450F^{™} (all available from Braskem S.A.).

An absorbent article component may comprise a bio-based content value from about 10% to about 100%, from about 25% to about 100%, from about 40% to about 100%, from about 50% to about 100%, from about 75% to about 100%, or from about 90% to about 100%, for example, using ASTM D6866-10, method B.

Components of the absorbent articles described herein may be recycled for other uses, whether they are formed, at least in part, from recyclable materials. Examples of absorbent article materials that may be recycled are nonwovens, films, fluff pulp, and superabsorbent polymers. The recycling process may use an autoclave for sterilizing the absorbent articles, after which the absorbent articles may be shredded and separated into different byproduct streams. Example byproduct streams may comprise plastic, superabsorbent polymer, and cellulose fiber, such as pulp. These byproduct streams may be used in the production of fertilizers, plastic articles of manufacture, paper products, viscose, construction materials, absorbent pads for pets or on hospital beds, and/or for other uses. Further details regarding absorbent articles that aid in recycling, designs of recycle friendly diapers, and designs of recycle friendly and bio-based component diapers, are disclosed in U.S. Pat. Appl. Publ. No. 2019/0192723, published on June 27, 2019.

### Test Methods

### TRAPEZOID TEAR STRENGTH TEST METHOD

The Trapezoid Tear Strength Test Method is used to characterize the tear strength of individual substrates, which may be a nonwoven substrate, and laminates, both with and without frangible pathways. The method is based on ASTM D5733-99, which is herein incorporated by reference. This method specifically pertains to the Trap Tear MD, as depicted in Fig. 3 of ASTM D5733-99, in which the tear propagation is generally along the lengthwise machine direction of the material being tested. In particular, the Trapezoid Tear Strength test method can be performed strictly in the MD direction or in a direction specified by angle of rotation relative to MD.

### Specimen identification and preparation

Substrates and laminates, either with or without frangible pathways, are generally excised as belts from finished articles. Side seams are first separated by hand, and belt portions are then separated from the chassis, optionally using a separation aid such as cryogenic freeze spray. Constituent substrates may be further excised from laminates via an additional step in which the substrate layers of a laminate and any elastics or other elements present are separated, again optionally using a separation aid such as cryogenic freeze spray. In some cases, substrates and laminates, either with or without frangible pathways, available as roll stock may be tested directly.

For substrates and laminates available as roll stock and to be tested free from frangible paths, at least six specimens are prepared as depicted and discussed for Trap Tear MD samples in section 7 of ASTM D5733-99. Substrates and laminates that are part of a finished article are separated from the article and similarly sampled. In general, the MD direction of belt substrates and laminates runs along the widthwise, lateral direction of a finished article and this is assumed unless there is clear evidence to the contrary, in which an alternative MD is noted. Specimens are then prepared as depicted and discussed for Trap Tear MD samples in section 7 of ASTM D5733-99, and such specimens are considered to be at 0° to the MD axis. Multiple articles are used as needed to collect the requisite number of specimens.

Specimen preparation is identical for substrates and laminates in the case for a nonzero rotation of the trapezoid away from the MD direction. In this case, the nonzero angle of rotation is noted and subsequently reported with the tear strength results.

If frangible pathways are to be interrogated on substrate or laminate on roll stock or harvested from finished articles, the frangible pathway is first identified and inspected. A portion of the frangible pathway of interest is identified. Trapezoidal specimens as depicted in Fig. 2 of ASTM D5733-99 are then cut such that the frangible pathway generally runs vertically on the trapezoid (along the 75 mm dimension) and generally centered horizontally such that the notch at the top of the trapezoid represents an initiation of tear along the frangible pathway. Six specimens are prepared in this way.

### Tensile tester usage and reporting

For the sample of interest, the prepared specimens are mounted in a tensile tester and pulled as is described in section 10 of ASTM D5733-99. Crosshead speed is 8.5 mm/s.

All specimens of the substrate or laminate sample (with or without a frangible pathway(s)) are tested, and for each specimen tested, load in Newtons (N) is plotted versus crosshead displacement (mm). In general, these plots will contain an initial region in which load increases, a plateau in which the force is fluctuating to some extent but neither increasing nor decreasing overall, and potentially an abrupt endpoint representing full breakage separation. For each specimen, the load is averaged in the plateau region (beginning at either the start of the plateau or at 6 mm of crosshead displacement, whichever occurs at greater crosshead displacement, and ending at the end of the end of the plateau region), and this is the tearing force for the specimen, recorded to the nearest 0.01 N. The average (arithmetic mean) among the tearing forces of all specimens is calculated and reported as the Pathway Tear Strength for those specimens having a frangible pathway and a Material Tear Strength for those specimens not having a frangible pathway. The pathway tear strength and the material tear strength are reported in Newtons to the nearest 0.01 N. For those specimens having a frangible pathway, the angle relative to the MD at which the tear was performed is also reported.

### SOFTNESS TEST METHOD

TS7 and TS750 values are measured using an EMTEC Tissue Softness Analyzer ("Emtec TSA") (Emtec Electronic GmbH, Leipzig, Germany) interfaced with a computer running Emtec TSA software (version 3.19 or equivalent). According to Emtec, the TS7 value correlates with the real material softness, while the TS750 value correlates with the felt smoothness/roughness of the material. The Emtec TSA comprises a rotor with vertical blades which rotate on the test sample at a defined and calibrated rotational speed (set by manufacturer) and contact force of 100 mN. Contact between the vertical blades and the test piece creates vibrations, which create sound that is recorded by a microphone within the instrument. The recorded sound file is then analyzed by the Emtec TSA software.

### Sample Preparation

Substrates and laminates are generally excised as belts from finished articles. Side seams are first separated by hand, and belt portions are then separated from the chassis, optionally using a separation aid such as cryogenic freeze spray. Constituent substrates may be further excised from laminates via an additional step in which the substrate layers of a laminate and any elastics or other elements present are separated, again optionally using a separation aid such as cryogenic freeze spray. In some cases, substrates and laminates, either with or without frangible pathways, available as roll stock may be tested directly. Test samples are prepared by cutting square or circular samples from a finished product. Test samples are cut to a length and width (or diameter if circular) of about 90 mm, and no greater than about 120 mm, in dimension. If the finished product has a discrete section of elastic region (i.e. elastic region is shorter in one or more dimensions than nonwoven facing-layers), a set of rectilinear specimens 76 mm ± 3 mm long in the primary stretch direction, and 100 mm ± 3 mm wide in the perpendicular direction is cut from the product part, with the elastic region centered in the rectilinear specimen. Test samples are selected to avoid creases or folds within the testing region, unless inherent to the sample such as corrugations. Prepare 8 substantially similar replicate samples for testing. Equilibrate all samples at TAPPI standard temperature and relative humidity conditions (23 °C ± 2 C° and 50 % ± 2 %) for at least 1 hour prior to conducting the TSA testing, which is also conducted under TAPPI conditions.

### Testing Procedure

Calibrate the instrument according to the manufacturer's instructions using the 1-point calibration method with Emtec reference standards ("ref.2 samples"). If these reference samples are no longer available, use the appropriate reference samples provided by the manufacturer. Calibrate the instrument according to the manufacturer's recommendation and instruction, so that the results will be comparable to those obtained when using the 1-point calibration method with Emtec reference standards ("ref.2 samples").

Mount the test sample into the instrument and ensure the sample is clamped into the TSA instrument properly with its first surface facing upwards. For samples with a discrete section of elastic region, ensure that the elastic region is centered below the Emtec vertical blades, and then perform the test according to the manufacturer's instructions. When complete, the software displays values for TS7 and TS750. Record each of these values to the nearest 0.01 dB V² rms. The test piece is then removed from the instrument and discarded. This testing is performed individually on the first surface of four of the replicate samples, and on the second surface of the other four replicate samples.

The four test result values for TS7 and TS750 from the first surface are averaged (using a simple numerical average); the same is done for the four test result values for TS7 and TS750 from the second surface. Report the individual average values and standard deviation of TS7 and TS750 for both the first and second surfaces on a particular test sample to the nearest 0.01 dB V² rms.

### AVERAGE DECITEX (AVERAGE-DTEX)

The Average Decitex Method is used to calculate the Average-Dtex on a length-weighted basis for elastic fibers present in an entire article, or in a specimen of interest extracted from an article. The decitex value is the mass in grams of a fiber present in 10,000 meters of that material in the relaxed state. The decitex value of elastic fibers or elastic laminates containing elastic fibers is often reported by manufacturers as part of a specification for an elastic fiber or an elastic laminate including elastic fibers. The Average-Dtex is to be calculated from these specifications if available. Alternatively, if these specified values are not known, the decitex value of an individual elastic fiber is measured by determining the cross-sectional area of a fiber in a relaxed state via a suitable microscopy technique such as scanning electron microscopy (SEM), determining the composition of the fiber via Fourier Transform Infrared (FT-IR) spectroscopy, and then using a literature value for density of the composition to calculate the mass in grams of the fiber present in 10,000 meters of the fiber. The manufacturer-provided or experimentally measured decitex values for the individual elastic fibers removed from an entire article, or specimen extracted from an article, are used in the expression below in which the length-weighted average of decitex value among elastic fibers present is determined.

The lengths of elastic fibers present in an article or specimen extracted from an article is calculated from overall dimensions of and the elastic fiber pre-strain ratio associated with components of the article with these or the specimen, respectively, if known. Alternatively, dimensions and/or elastic fiber pre-strain ratios are not known, an absorbent article or specimen extracted from an absorbent article is disassembled and all elastic fibers are removed. This disassembly can be done, for example, with gentle heating to soften adhesives, with a cryogenic spray (e.g., Quick-Freeze, Miller-Stephenson Company, Danbury, CT), or with an appropriate solvent that will remove adhesive but not swell, alter, or destroy elastic fibers. The length of each elastic fiber in its relaxed state is measured and recorded in millimeters (mm) to the nearest mm.

### Calculation of Average-Dtex

For each of the individual elastic fibers *fᵢ* of relaxed length *Lᵢ* and fiber decitex value *dᵢ* (obtained either from the manufacturer's specifications or measured experimentally) present in an absorbent article, or specimen extracted from an absorbent article, the Average-Dtex for that absorbent article or specimen extracted from an absorbent article is defined as: $Average - Dtex = \frac{{\sum }_{i = 1}^{n} \left({L}_{i}\times {d}_{i}\right)}{{\sum }_{i = 1}^{n} {L}_{i}}$ where n is the total number of elastic fibers present in an absorbent article or specimen extracted from an absorbent article. The Average-Dtex is reported to the nearest integer value of decitex (grams per 10 000 m).

If the decitex value of any individual fiber is not known from specifications, it is experimentally determined as described below, and the resulting fiber decitex value(s) are used in the above equation to determine Average-Dtex.

### Experimental Determination of Decitex Value for a Fiber

For each of the elastic fibers removed from an absorbent article or specimen extracted from an absorbent article according to the procedure described above, the length of each elastic fiber *Lₖ* in its relaxed state is measured and recorded in millimeters (mm) to the nearest mm. Each elastic fiber is analyzed via FT-IR spectroscopy to determine its composition, and its density *ρₖ* is determined from available literature values. Finally, each fiber is analyzed via SEM. The fiber is cut in three approximately equal locations perpendicularly along its length with a sharp blade to create a clean cross-section for SEM analysis. Three fiber segments with these cross sections exposed are mounted on an SEM sample holder in a relaxed state, sputter coated with gold, introduced into an SEM for analysis, and imaged at a resolution sufficient to clearly elucidate fiber cross sections. Fiber cross sections are oriented as perpendicular as possible to the detector to minimize any oblique distortion in the measured cross sections. Fiber cross sections may vary in shape, and some fibers may consist of a plurality of individual filaments. Regardless, the area of each of the three fiber cross sections is determined (for example, using diameters for round fibers, major and minor axes for elliptical fibers, and image analysis for more complicated shapes), and the average of the three areas *aₖ* for the elastic fiber, in units of micrometers squared (µm²), is recorded to the nearest 0.1 µm². The decitex *dₖ* of the kth elastic fiber measured is calculated by: ${d}_{k} = 10 000 m \times {a}_{k} \times {ρ}_{k} \times {10}^{- 6}$ where *dₖ* is in units of grams (per calculated 10,000 meter length), *aₖ* is in units of µm², and *ρₖ* is in units of grams per cubic centimeter (g/cm³). For any elastic fiber analyzed, the experimentally determined *Lₖ* and *dₖ* values are subsequently used in the expression above for Average-Dtex.

### AVERAGE-STRAND-SPACING

Using a ruler calibrated against a certified NIST ruler and accurate to 0.5 mm, measure the distance between the two distal strands within a section to the nearest 0.5 mm, and then divide by the number of strands in that section - 1 $Average - Strand - Spacing = d / \left(n-1\right)$ where n>1
report to the nearest 0.1 mm.

### AVERAGE-PRE-STRAIN

The Average-Pre-Strain of a specimen are measured on a constant rate of extension tensile tester (a suitable instrument is the MTS Insight using Testworks 4.0 Software, as available from MTS Systems Corp., Eden Prairie, MN) using a load cell for which the forces measured are within 1% to 90% of the limit of the cell. Articles are conditioned at 23 °C ± 2 C° and 50% ± 2% relative humidity for 2 hours prior to analysis and then tested under the same environmental conditions.

Program the tensile tester to perform an elongation to break after an initial gage length adjustment. First raise the cross head at 10 mm/min up to a force of 0.05N. Set the current gage to the adjusted gage length. Raise the crosshead at a rate of 100 mm/min until the specimen breaks (force drops 20% after maximum peak force). Return the cross head to its original position. Force and extension data is acquired at a rate of 100 Hz throughout the experiment.

Set the nominal gage length to 40 mm using a calibrated caliper block and zero the crosshead. Insert the specimen into the upper grip such that the middle of the test strip is positioned 20 mm below the grip. The specimen may be folded perpendicular to the pull axis, and placed in the grip to achieve this position. After the grip is closed the excess material can be trimmed. Insert the specimen into the lower grips and close. Once again, the strip can be folded, and then trimmed after the grip is closed. Zero the load cell. The specimen should have a minimal slack but less than 0.05 N of force on the load cell. Start the test program.

From the data construct a Force (N) verses Extension (mm). The Average-Pre-Strain is calculated from the bend in the curve corresponding to the extension at which the nonwovens in the elastic are engaged. Plot two lines, corresponding to the region of the curve before the bend (primarily the elastics), and the region after the bend (primarily the nonwovens). Read the extension at which these two lines intersect, and calculate the % Pre-Strain from the extension and the corrected gage length. Record as % Pre-strain 0.1%. Calculate the arithmetic mean of three replicate samples for each elastomeric laminate and Average-Pre-Strain to the nearest 0.1%.

## Claims

1. An absorbent article comprising:
a chassis (102) comprising a topsheet (138), a backsheet (136), and an absorbent core (142) positioned between the topsheet (138) and the backsheet (136), the chassis (102) comprising a longitudinally extending first side edge and a longitudinally extending second side edge laterally separated from the first side edge by a first end edge and a second end edge longitudinally separate from the first end edge;
a first belt (106) joined to a portion of the chassis (102), wherein the first belt (106) comprises a first substrate (162), wherein the first substrate (162) comprises a first frangible pathway,
wherein the first substrate (162) has a material tear strength according to the Trapezoid Tear Strength test method and a material basis weight, wherein the first frangible pathway has a pathway tear strength according to the Trapezoid Tear Strength test method, and
wherein the material tear strength of the first substrate (162) is greater than the pathway tear strength of the first frangible pathway,
wherein the absorbent article is **characterised in that** at least a portion of the first frangible pathway extends at a pathway angle of less than 90 degrees from a lateral centerline of the first belt (106),
wherein the material basis weight of the first substrate (162) is from about 8 gsm to about 30 gsm,
wherein the pathway tear strength of the first frangible pathway is greater than 8 N, and the material tear strength of the first substrate (162) is at least 2 N greater than the pathway tear strength of the first frangible pathway, and
wherein the material tear strength of the first substrate (162) divided by the material basis weight of the first substrate (162) is greater than 0.2 N/gsm.

2. The absorbent article according to claim **1,** wherein the material tear strength of the first substrate (162) divided by the material basis weight of the first substrate (106) is greater than 0.3 N/gsm.

3. The absorbent article according to any one of the preceding claims, wherein the first frangible pathway has a pathway angle of from about 0 degrees to about 75 degrees from the lateral centerline (126a) of the first belt (106).

4. The absorbent article according to claim 3, wherein the pathway angle is from about 15 degrees to about 45 degrees from the lateral centerline (126a) of the first belt (106).

5. The absorbent article according to claim 1, wherein the first belt (106) comprises a second substrate (164) having a second frangible pathway, wherein the first substrate (162) and the second substrate (164) are joined to form the first belt (106).

6. The absorbent article according to claim 5, wherein the second substrate (164) has a second material tear strength according to the Trapezoid Tear Strength test method, wherein the second substrate (164) has a second material basis weight, and wherein the second material tear strength divided by the second material basis weight is greater than 0.2 N/gsm.

7. The absorbent article according to claim 5, wherein the first frangible pathway and the second frangible pathway are aligned.

8. The absorbent article according to claim 6, wherein the material basis weight and the second material basis weight are different.

9. The absorbent article according to claim 1, wherein the difference between the material tear strength and the pathway tear strength is a tear strength delta, and wherein the tear strength delta divided by the material basis weight is greater than 0.2 N/gsm.

10. The absorbent article according to claim 9, wherein the tear strength delta divided by the material basis weight is greater than 0.3 N/gsm.

11. The absorbent article according to claim 1, wherein the difference between the material tear strength and the pathway tear strength is at least 6N.

12. The absorbent article according to claim 1, wherein the first substrate (162) is a nonwoven.

## Patentansprüche

1. Absorptionsartikel, umfassend:
eine Grundeinheit (102), umfassend eine Oberschicht (138), eine Unterschicht (136) und einen Absorptionskern (142), der zwischen der Oberschicht (138) und der Unterschicht (136) angeordnet ist, die Grundeinheit (102) umfassend einen sich in Längsrichtung erstreckenden ersten Seitenrand und einen sich in Längsrichtung erstreckenden zweiten Seitenrand, der von dem ersten Seitenrand durch einen ersten Endrand und einen zweiten Endrand lateral getrennt ist, der in Längsrichtung von dem ersten Endrand getrennt ist;
einen ersten Riemen (106), der mit einem Teil der Grundeinheit (102) gefaltet ist, wobei der erste Riemen (106) ein erstes Substrat (162) umfasst, wobei das erste Substrat (162) einen ersten reißbaren Pfad umfasst,
wobei das erste Substrat (162) eine Materialreißfestigkeit gemäß dem Trapez-Reißfestigkeitsprüfverfahren und ein Materialbasisgewicht aufweist, wobei der erste reißbare Pfad eine Pfadreißfestigkeit gemäß dem Trapez-Reißfestigkeitsprüfverfahren aufweist und
wobei die Materialreißfestigkeit des ersten Substrats (162) größer als die Pfadreißfestigkeit des ersten reißbaren Pfads ist,
wobei der Absorptionsartikel **dadurch gekennzeichnet ist, dass** sich wenigstens ein Teil des ersten reißbaren Pfads in einem Pfadwinkel von weniger als 90 Grad von einer Querachse des ersten Riemens (106) erstreckt,
wobei das Materialbasisgewicht des ersten Substrats (162) von etwa 8 g/m² bis etwa 30 g/m² beträgt,
wobei die Pfadreißfestigkeit des ersten reißbaren Pfads größer als 8 N ist, und die Materialreißfestigkeit des ersten Substrats (162) wenigstens 2 N größer als die Pfadreißfestigkeit des ersten reißbaren Pfads ist und
wobei die Materialreißfestigkeit des ersten Substrats (162) dividiert durch das Materialbasisgewicht des ersten Substrats (162) größer als 0,2 N/g/m² ist.

2. Absorptionsartikel nach Anspruch 1, wobei die Materialreißfestigkeit des ersten Substrats (162) dividiert durch das Materialbasisgewicht des ersten Substrats (106) größer als 0,3 N/g/m² ist.

3. Absorptionsartikel nach einem der vorstehenden Ansprüche, wobei der erste reißbare Pfad einen Pfadwinkel von etwa 0 Grad bis etwa 75 Grad von der Querachse (126a) des ersten Riemens (106) aufweist.

4. Absorptionsartikel nach Anspruch 3, wobei der Pfadwinkel von der Querachse (126a) des ersten Riemens (106) etwa 15 Grad bis etwa 45 Grad beträgt.

5. Absorptionsartikel nach Anspruch 1, wobei der erste Riemen (106) ein zweites Substrat (164) umfasst, das einen zweiten reißbaren Pfad aufweist, wobei das erste Substrat (162) und das zweite Substrat (164) gefaltet sind, um den ersten Riemen (106) auszubilden.

6. Absorptionsartikel nach Anspruch 5, wobei das zweite Substrat (164) eine zweite Materialreißfestigkeit gemäß dem Trapez-Reißfestigkeitsprüfverfahren aufweist, wobei das zweite Substrat (164) ein zweites Materialbasisgewicht aufweist, und wobei die zweite Materialreißfestigkeit dividiert durch das zweite Materialbasisgewicht größer als 0,2 N/g/m² ist.

7. Absorptionsartikel nach Anspruch 5, wobei der erste reißbare Pfad und der zweite reißbare Pfad ausgerichtet sind.

8. Absorptionsartikel nach Anspruch 6, wobei das Materialbasisgewicht und das zweite Materialbasisgewicht unterschiedlich sind.

9. Absorptionsartikel nach Anspruch 1, wobei die Differenz zwischen der Materialreißfestigkeit und der Pfadreißfestigkeit ein Reißfestigkeitsdelta ist, und wobei das Reißfestigkeitsdelta dividiert durch das Materialbasisgewicht größer als 0,2 N/g/m² ist.

10. Absorptionsartikel nach Anspruch 9, wobei das Reißfestigkeitsdelta dividiert durch das Materialbasisgewicht größer als 0,3 N/g/m² ist.

11. Absorptionsartikel nach Anspruch 1, wobei die Differenz zwischen der Materialreißfestigkeit und der Pfadreißfestigkeit wenigstens 6 N beträgt.

12. Absorptionsartikel nach Anspruch 1, wobei das erste Substrat (162) ein Vlies ist.

## Revendications

1. Article absorbant comprenant :
un châssis (102) comprenant une feuille de dessus (138), une feuille de fond (136) et une âme absorbante (142) positionnée entre la feuille de dessus (138) et la feuille de fond (136), le châssis (102) comprenant un premier bord latéral s'étendant longitudinalement et un second bord latéral s'étendant longitudinalement séparé latéralement du premier bord latéral par un premier bord d'extrémité et un second bord d'extrémité séparé longitudinalement du premier bord d'extrémité ;
une première ceinture (106) reliée à une partie du châssis (102), dans lequel la première ceinture (106) comprend un premier substrat (162), dans lequel le premier substrat (162) comprend une première voie frangible,
dans lequel le premier substrat (162) a une résistance au déchirement de matériau selon la méthode d'essai de résistance au déchirement trapézoïdal et une masse surfacique de matériau, dans lequel la première voie frangible a une résistance au déchirement de voie selon la méthode d'essai de résistance au déchirement trapézoïdal, et
dans lequel la résistance au déchirement du matériau du premier substrat (162) est supérieure à la résistance au déchirement de voie de la première voie frangible,
dans lequel l'article absorbant est **caractérisé en ce qu'**au moins une partie de la première voie frangible s'étend selon un angle de voie inférieur à 90 degrés à partir d'une ligne médiane latérale de la première ceinture (106),
dans lequel la masse surfacique du premier substrat (162) va d'environ 8 g/m² à environ 30 g/m²,
dans lequel la résistance au déchirement de voie de la première voie frangible est supérieure à 8 N, et la résistance au déchirement de matériau du premier substrat (162) est supérieure d'au moins 2 N à la résistance au déchirement de voie de la première voie frangible, et
dans lequel la résistance au déchirement de matériau du premier substrat (162) divisée par la masse surfacique de matériau du premier substrat (162) est supérieure à 0,2 N/g/m².

2. Article absorbant selon la revendication 1, dans lequel la résistance au déchirement de matériau du premier substrat (162) divisée par la masse surfacique de matériau du premier substrat (106) est supérieure à 0,3 N/g/m².

3. Article absorbant selon l'une quelconque des revendications précédentes, dans lequel la première voie frangible a un angle de voie allant d'environ 0 degré à environ 75 degrés à partir de la ligne médiane latérale (126a) de la première ceinture (106).

4. Article absorbant selon la revendication 3, dans lequel l'angle de voie est d'environ 15 degrés à environ 45 degrés par rapport à la ligne médiane latérale (126a) de la première ceinture (106).

5. Article absorbant selon la revendication 1, dans lequel la première ceinture (106) comprend un second substrat (164) ayant une seconde voie frangible, dans lequel le premier substrat (162) et le second substrat (164) sont joints pour former la première ceinture (106).

6. Article absorbant selon la revendication 5, dans lequel le second substrat (164) a une seconde résistance au déchirement de matériau selon la méthode d'essai de résistance au déchirement trapézoïdal, dans lequel le second substrat (164) a une seconde masse surfacique de matériau, et dans lequel la seconde résistance au déchirement de matériau divisée par le second poids de masse surfacique de matériau est supérieur à 0,2 N/g/m².

7. Article absorbant selon la revendication 5, dans lequel la première voie frangible et la seconde voie frangible sont alignées.

8. Article absorbant selon la revendication 6, dans lequel la masse surfacique de matériau et la seconde masse surfacique de matériau sont différentes.

9. Article absorbant selon la revendication 1, dans lequel la différence entre la résistance au déchirement de matériau et la résistance au déchirement de voie est un delta de résistance au déchirement, et dans lequel le delta de résistance au déchirement divisé par la masse surfacique de matériau est supérieur à 0,2 N/g/m².

10. Article absorbant selon la revendication 9, dans lequel le delta de résistance au déchirement divisé par la masse surfacique de matériau est supérieur à 0,3 N/g/m².

11. Article absorbant selon la revendication 1, dans lequel la différence entre la résistance au déchirement de matériau et la résistance au déchirement de voie est d'au moins 6 N.

12. Article absorbant selon la revendication 1, dans lequel le premier substrat (162) est un non-tissé.
